# EUROPEAN PATENT SPECIFICATION

(11) **EP 1 605 039 B1**
(45) Date of publication and mention of the grant of the patent: **23.04.2008**
(21) Application number: 04716785.3
(22) Date of filing: 03.03.2004
(51) Int. Cl.: C12N 5/06, C12N 5/08, A61K 35/12, A61P 37/00, A61P 37/06, A61P 37/08

(54) **DENDRITIC CELL PRESENTING ALPHA-GLYCOSYLCERAMIDE DERIVATIVE AND A NTIGEN AND USABLE IN SUPPRESSING IMMUNE RESPONSE**
DENDRITISCHE ZELLE MIT ALPHA-GLYCOSYLCERAMID-DERIVAT UND ANTIGEN ZUR VERWENDUNG BEI DER UNTERDRÜCKUNG VON IMMUNREAKTIONEN
CELLULE DENDRITIQUE PRESENTANT DES DERIVES DE ALPHA-GLYCOSYLCERAMIDE ET UN ANTIGENE ET UTILISABLE DANS LA SUPPRESSION DE LA REPONSE IMMUNITAIRE

(30) Priority: 03.03.2003 JP 2003056109
(43) Date of publication of application: 14.12.2005
(73) Proprietor: Kirin Pharma Kabushiki Kaisha, Shibuya-ku Tokyo (JP)
(72) Inventor: SERIZAWA, Isao, Takasaki-shi, Gunma 3701295 (JP); YAMAGUCHI, Yasunori, Shibuya-ku, Tokyo 3701295 (JP); EHARA, Hiromi, Takasaki-shi, Gunma 3701295 (JP)
(74) Representative: HOFFMANN EITLE
(86) International application number: PCT/JP2004/002621
(87) International publication number: WO 2004/078957

(56) References cited:
- EP-A- 0 988 860
- WO-A2-02/056830
- NIEDA M ET AL: "Dendritic cells rapidly undergo apoptosis in vitro following culture with activated CD4+ Valpha24 natural killer T cells expressing CD40L" IMMUNOLOGY, vol. 102, no. 2, February 2001 (2001-02), pages 137-145, XP002364637 ISSN: 0019-2805
- NIEDA M ET AL: "ACTIVATION OF HUMAN VALPHA24NKT CELLS BY ALPHA-GLYCOSYLCERAMIDE IN A CD1D-RESTRICTED AND VALPHA24TCR-MEDIATED MANNER" HUMAN IMMUNOLOGY, NEW YORK, NY, US, vol. 60, no. 1, January 1999 (1999-01), pages 10-19, XP001146056 ISSN: 0198-8859
- TAKAHASHI T ET AL: "Analysis of human V alpha 24+ CD4+ NKT cells activated by alpha-glycosylceramide-pulsed monocyte-derived dendritic cells." JOURNAL OF IMMUNOLOGY (BALTIMORE, MD. : 1950) 1 MAY 2000, vol. 164, no. 9, 1 May 2000 (2000-05-01), pages 4458-4464, XP002364638 ISSN: 0022-1767
- DATABASE WPI Section Ch, Week 200250 Derwent Publications Ltd., London, GB; Class B04, AN 2002-471727 XP002364641 & WO 02/42433 A1 (KIRIN BREWERY KK) 30 May 2002 (2002-05-30)
- DATABASE WPI Section Ch, Week 200334 Derwent Publications Ltd., London, GB; Class B03, AN 2003-357606 XP002364642 & JP 2002 284692 A (KIRIN BREWERY KK) 3 October 2002 (2002-10-03)
- NISHI, N. ET AK: 'Synergistic effect of KRN7000 with interleukin-15, -7 and -2 on the expansion of human V alpha 24+V beta 11+ T cells in vitro' HUM IMMUNOL, vol. 61, no. 4, 2000, pages 357 - 365, XP001146058
- LUTZ, M.B. ET AL: 'Immature dendritic cells generated with low doses of GM-GSF in the absence of IL-4 are maturation resistant and prolong allograft survival in vivo' EUR J IMMUNOL vol. 30, no. 7, 2000, pages 1813 - 1822, XP001155683
- LUTZ, M.B. ET AL: 'Immature, semi-mature and fully mature dendritic cells: which signals induce tolerance or immunity?' TRENDS IMMUNOL vol. 23, no. 9, 2002, pages 445 - 449, XP004377436

## Description

The present invention relates to the medical use for the treatment of diseases including various autoimmune diseases, graft rejection, graft versus host disease, and various allergic diseases of a dendritic cell presenting an α-glycosylceramide derivative and an antigen.

KRN7000 (Tetrahedron Lett., 34: 5591-5592, 1993; Tetrahedron Lett., 34: 5593-5596, 1993; Tetrahedron, 50: 2771-2784, 1994) synthesized as one of derivatives of agelasphin derived from a marine sponge has been identified as a ligand of an invariable T-cell receptor (TCR) expressed in natural killer T (NKT) cells (Science, 278:1626-1629, 1997; J. Exp. Med., 188:1521-1528 1998; J. Exp. Med. 188:1529-1534, 1998).

α-Glycosylceramide derivatives including KRN7000 are sphingoglycollpids in which hydrophilic saccharides such as galactose and glucose and hydrophobic ceramides comprising fatty acid-sphingosine base are linked in an alpha configuration, and are reported to show potent anti-tumorous activities by stimulating NKT cells as a ligand thereof (Oncol. Res., 7:529-534, 1995; Cancer Res., 58:1202-1207, 1998). The α-glycosylceramide derivative as a glycolipid antigen is incorporated into antigen-presenting cells (APC) including dendritic cells (DC), then processed via no TAP (transporter associated with antigen processing) pathway which is known to be responsible for the processing of protein antigens, and presented on APC membrane by major histocompatibility antigen (MHC) class Ib-like non-polymorphic molecule CD1d. The NKT cells are activated by the recognition of α-glycosylceramide derivative presented by CD1d on APC by TCR having no diversity on the membrane of said NKT cells. It has been reported that human NKT cells can be cultured efficiently with human monocyte-derived DC having the α-glycosylceramide derivative incorporated therein on the basis of the above principle (Hum. Immunol., 60:10-19, 1999). It has also been reported that interleukin-7 (IL-7) or IL-15 proliferate in vitro NKT cells synergically together with the α-glycosylceramide derivative (Hum. Immunol., 61:357-365, 1999).

It has been revealed that the NKT cells are intermediate TCR cells (TCR^{int} cells) which express moderate level of the TCR, and are similar to natural killer (NK) cells in that these cells show large granular lymphocyte (LGL)-like form, regularly express IL-2 receptor β chain, and have perforin granules, but are the cell group clearly different from the NK cells in that the NKT cells have TCR (Watanabe, H. et al., J. Immunol., 155, 2972, 1995). In addition, it has been demonstrated that NK1.1⁺TCR^{Int} (NKT) cell which expresses NK1.1 among TCR^{Int} cells activated by interleukin-12 (IL-12) is an important effector cell for suppressing circulatory metastasis of tumor into liver or lung in mice (Hashimoto, W. et al., J. Immunol., 154, 4333, 1995; Anzai, R. et al., Immunol., 88, 82, 1996). Thus, the NKT cells are regarded as the cell which plays an important role in the elimination of cancer cells, parasites, protozoans, and intra-cellular infective bacteria such as listeria and tubercle bacillus (Seki, S. et al., Clin. Immunol., 28, 1069, 1996). Furthermore, the NKT cells are also known as the cells closely responsible for acute rejection in marrow grafts (Yankelevich, B. et al., J. Immunol., 142, 3423, 1989), control of producing IgE antibody by the control of differentiation of Th1/Th2 of helper T cells (Yoshimoto, T. et al., J. Exp. Med., 179, 1285, 1994), and the like. As described above, the NKT cells are the cell group which has been recently paid attention as a new cell group responsible for a variety of biomechanisms.

Va14⁺NKT cell is a subset of the NKT cells. Many of the Va14⁺NKT cells express Vα14Jα281mRNA, and its protein functions as TCR α chain. It has been recently demonstrated that the Vα14⁺NKT cells are closely responsible for the development of autoimmune disease. That is to say, MRL/I-*lpr*/*lpr* mouse is the model mouse of autoimmune disease (human systemic lupus erythematosus) which causes the accumulation of abnormal lymphocytes at 17-20 weeks after birth, and it has been found in this mouse that Vα14⁺NKT cells decrease selectively prior to the development of autoimmune disease (Mieza, M.A. et al., J. Immunol., 156, 4035, 1996). It has been reported that similar phenomenon is also observed in gld mice and (NZBxNZW)F1 mice, and the Vα14⁺NKT cells are closely involved in the development of autoimmune disease (Makino, Y. et al., Clin. Immunol., 28, 1487, 1996). In addition, interestingly, analogous phenomenon has also been observed in human cases. That is to say, it has been observed that a human homologue Vα24JαQα chain which is homologous to murine Vα14Jα281 chain is present in 20-50% of CD4-/CD8-T cells in peripheral blood of healthy individuals but it is extremely decreased in scleroderma patients (Sumida, T. et al., J. Exp. Med., 182, 1163, 1995). As the above, it is known that murine Vα14⁺NKT cells or human Vα24JαQαT cells are responsible for a variety of autoimmune diseases, of which etiological genes or genetic backgrounds are different. Accordingly, IL-12 having an effect of activating the NKT cells, as described above, had been expected to be a pharmaceutical agent of autoimmune diseases such as systemic lupus erythematosus (SLE) and systemic scleroderma (SSc). However, it has been observed that abnormal lymphocytes (CD3⁺ B220⁺ CD4 CD8 double negative T cells) was remarkably increased in spleen and lymph nodes of MRL/I-Ipr/Ipr mice injected with IL-12 as compared with non-injected mice (Takeki Tsutsui et al., Proceedings of Annual Meeting of the Japanese Society for Immunology, 347, 1996).

On the other hand, β-galactosylceramides, β-glucosylceramides and the like wherein a various saccharide is attached to ceramide by β-bonding are present in living bodies (Svennerholm, L. et al., Biochem. Blophys. Acta, 280, 626, 1972; Karlsson, K.-A. et al., Biochim. Biophys. Acta, 316, 317, 1973). In addition, it is known that α-galactosylceramides have remarkable immune-activating effect and antitumor activity (Morita, M. et al., J. Med. Chem., 38, 2176, 1995), and that these activities of α-galactosyiceramides and α-glucosylceramides are much more potent than those of β-galactosylceramides and β-glucosylceramides (Motoki, K. et al., Biol. Pharm. Bull., 18, 1487, 1995). Further, it is also known that the compound having α-glucosylceramide structure possesses radioprotective activities on its *in vivo* administration (Motokl, K. et al., Bioorg. Med. Chem. Lett., 5, 2413, 1995), inhibitory activities in B16 melanoma lung methastasis (Kobayashi, E. et al., Oncology Res., 7, 529, 1995), and Colon 26 colon cancer or EL-4 T lymphoma liver methastasis (Kazuhiro Motoki et al., Proceedings of Annual Meeting of The Japanese Cancer Association, 523, 1996), as well as activities of increasing platelet and white blood cell (Motoki, K. et al., Biol. Pharm. Bull., 19, 952, 1996). It has also been reported that the α-glycosylceramide derivative is effective for the treatment of type I diabetes in NOD mice (Hong, S. et al., Nat. Med., 7, 1052, 2001; Sharif, S. et al., Nat. Med., 7, 1057, 2001), EAE (Singh, A. K. et al., J. Exp. Med., 194, 1801, 2001), or an autoimmune disease model in mice.

In addition, the α-glycosylceramide derivative is presented in CD1d highly expressed on DC. It has been reported that DC treated *ex vivo* with the α-glycosylceramide derivative shows potent antitumorous activities in mice on administration thereof. In this phenomenon, the α-glycosylceramide derivative presented on DC efficiently activates NKT cell fraction and accelerate the production of cytokines such as IFN-γ or IL-4.

DC is known to activate both CD4⁺ T cells and CD8⁺ T cells (Inaba et al., J. Exp. Med., 172, 631, 1990; Inaba et al., Int. Rev. Immunol., 6, 197, 1990; Porgador and Gilboa, J. Exp. Med., 182, 255, 1995). DC is distributed in an immature form in non-lymphatic tissues and maintains potent activity of processing antigen in this form. After processing antigen, DC migrates to lymph nodes to which it belongs, and presents the antigen for T cells in the T cell area and activates the T cells.

DC is converted to immune-activated state by inflammatory stimulus. This process is referred to as "maturation" of DC, in which phenotype and functions of DC is changed (including the increase of expression level of costimulatory molecules, adhesion molecules and specific chemokines/chemokine receptors).

It has been recently reported that immune reaction is not induced and immune suppression occurs in the case where immature DC is employed (USP 5,871,728; Lutz et al., Eur. J. Immunol., Jul; 30(7): 1813-22, 2000). In that case, it has been shown that immunological tolerance is induced by the pretreatment with DC, and it has been described by Steinman et al. that tolerance is induced also on the once established immune by the repeated treatments with immature DC (WO 02/056830).

It is also known with regard to DC subjected to maturation treatment that suppressed immune reaction is established when the semi-matured DC is injected intravenously (Gerald Schuler et al. TRENDS in immunology Vol. 23, No9, September 2002). According to this document, the semi-maturation of DC is induced depending on the cytokines used for the maturation process, whereby antigen-specific immune suppression is established, and thus a therapeutic method which is effective for autoimmune disease is expected to be developed.

However, it has not been reported that immune response is strongly suppressed when DC was treated with an antigen and the α-glycosylceramide derivative simultaneously. It has not also been reported that immune response is strongly suppressed when DC treated with an antigen and DC treated with the α-glycosylceramide derivative are used in combination.

The present inventors have found that the development of experimental autoimmune encephalomyelitis (EAE) as a multiple sclerosis model is remarkably suppressed by injecting dendritic cells (DC) treated with a myelin antigen and an α-glycosylceramide derivative. This effect is observed by injecting DC treated with both of the myelin antigen and the α-glycosylceramide derivative, but not by injecting DC treated with either the myelin antigen or the α-glycosylceramide derivative alone. The suppressive effect is superior to the effect of the systemic administration of the α-glycosylceramide derivative alone.

The present inventors have also found that the symptoms of type II collagen-induced arthritis (CIA) as a model of rheumatoid arthritis is remarkably suppressed by injecting DC treated with bovine type II collagen and an α-glycosylceramide derivative.

Further, the present inventors have found that the induction of specific antigen-specific cytotoxic CD8 positive-T-lymphocyte (CTL) is suppressed by injecting DC treated with said antigen and the α-glycosylceramide derivative.

Therefore, the present inventors have found that the immune response of CD4 positive T cells or CD8 positive T cells can be suppressively regulated by injecting DC treated with an antigen and an α-glycosylceramide derivative.

In addition, the present inventors have found that the immune response of CD4 positive T cells or CD8 positive T cells can be suppressively regulated by injecting DC treated with an antigen and DC treated with an α-glycosylceramide derivative in combination. The present invention is based on these findings.

Accordingly, the objects of the present invention is the medical use of a dendritic cell capable of suppressing T cell-dependent immune response specific for an antigen (a producing method thereof, is also described), and the medical use of a cell mixture capable of suppressing T cell-dependent immune response specific for an antigen. A pharmaceutical composition comprising said dendritic cell or said cell mixture is also described.

The dendritic cell is a dendritic cell capable of suppressing T cell-dependent immune response specific for an antigen, which presents antigen fragment of said antigen which is presented against T cell in said immune response, and α-glycosylceramide derivative.

Furthermore, the producing method is a method of producing a dendritic cell capable of suppressing T cell-dependent immune response specific for an antigen, which presents antigen fragment of said antigen which is presented against T cell in said immune response, and α-glycosylceramide derivative, comprising a step of culturing dendritic cell in the presence of α-glycosylceramide derivative.

In addition, the cell mixture is a cell mixture capable of suppressing T cell-dependent immune response specific for an antigen, which comprises dendritic cells presenting at least antigen fragment of said antigen which is presented against T cell in said immune response, and dendritic cells presenting at least α-glycosylceramide derivative.

Moreover, the pharmaceutical composition is a pharmaceutical composition for the treatment of disease induced by T cell-dependent immune response, comprising the dendritic cell or the cell mixture.

A new method for the treatment of a variety of diseases induced by T cell-dependent immune response is presented.
Fig. 1 illustrates the suppression of the development of EAE by administration of DC which has been loaded with KRN7000 and MOG peptide antigen.
Fig. 2 illustrates the suppression of decrease of body weight involved in the development of EAE by administration of DC which has been loaded with KRN7000 and MOG peptide antigen.
Fig. 3 illustrates anti-tumor activities by administration of DC which has been loaded with tumor antigen, and the suppression of the anti-tumor activities by administration of DC which has been loaded with KRN7000 and tumor antigen.
Fig. 4 illustrates the inductive production of antigen specific interferon y by administration of DC which has been loaded with tumor antigen, and suppressed production of the interferon by DC which has been loaded with KRN7000 and tumor antigen.
Fig. 5 illustrates the suppression of type II collagen-induced arthritis by administration of DC which has been loaded with KRN7000 and antigen.
Fig. 6 illustrates the suppression of decrease of body weight involved in the development of type II collagen-induced arthritis by administration of DC which has been loaded with KRN7000 and antigen.
Fig. 7 illustrates the suppression of EAE by administration of DC which has been loaded with both of KRN7000 and MOG peptide antigen, or by administration of a cell mixture of DC which has been loaded with KRN7000 alone and DC which has been loaded with MOG peptide antigen alone, after induction of EAE.
Fig. 8 illustrates the suppression of decrease of body weight involved in the development of EAE by administration of DC which has been loaded with both of KRN7000 and MOG peptide antigen, or by administration of a cell mixture of DC which has been loaded with KRN7000 alone and DC which has been loaded with MOG peptide antigen alone.
Fig. 9 illustrates the suppression of the development of delayed hypersensitivity caused by OVA as an antigen by administration of DC which has been loaded with both of KRN7000 and OVA antigen.

On the dendritic cell (DC) an antigen fragment of an antigen which is presented against a T cell in T cell-dependent immune response specific for the antigen, and an α-glycosylceramide derivative are presented. Thereby, the DC is able to suppress T cell-dependent immune response specific for the antigen. The T cell is preferably, but not limited to, CD4 positive T cell and/or CD8 positive T cell.

The cell mixture comprises dendritic cell presenting at least an antigen fragment of an antigen which is presented against a T cell in T cell-dependent immune response specific for the antigen, and dendritic cell presenting at least an α-glycosylceramide derivative. Thereby, the cell mixture is able to suppress T cell-dependent immune response specific for the antigen. The T cell is preferably, but not limited to, CD4 positive T cell and/or CD8 positive T cell.

In one embodiment, the cell mixture comprises dendritic cells presenting the antigen fragment (hereinafter referred to as "specific antigen-presenting DC"), and dendritic cells presenting the α-glycosylceramide derivative (hereinafter referred to as "α-glycosylceramide derivative-presenting DC"). In another embodiment, the cell mixture comprises the specific antigen-presenting DC and/or the α-glycosylceramide derivative-presenting DC, and the DC.

DCs contained in the cell mixture are also able to suppress T cell-dependent immune response specific for an antigen even if these DCs are used in combination, not in the form of admixture. Therefore, in another aspect, the present invention provides the medical use of a combination of dendritic cells capable of suppressing T cell-dependent immune response specific for an antigen, which comprises dendritic cells presenting at least antigen fragment of said antigen which is presented against T cell in said immune response, and dendritic cells presenting at least α-glycosylceramide derivative. The T cell is preferably, but not limited to, CD4 positive T cell and/or CD8 positive T cell.

In one embodiment, the combination comprises the specific antigen-presenting DC and the α-glycosylceramide derivative-presenting DC. In another embodiment, the combination comprises the specific antigen-presenting DC and/or the a-glycosylceramide derivative-presenting DC, and the DC according to the present invention.

The α-glycosylceramide derivative is preferably, but not limited to, the compound represented by the following formula (I), or a salt or solvate thereof: 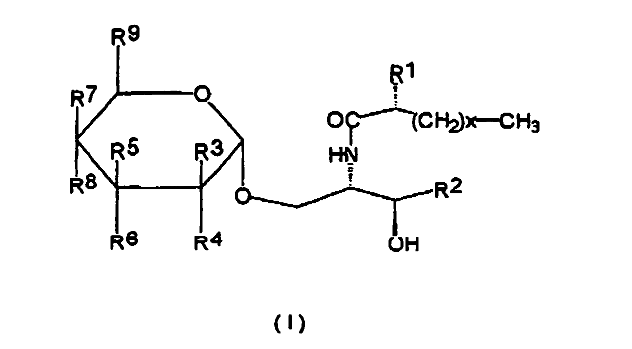 wherein
R¹ represents H or OH,
X represents an Integer between 7 and 27,
R² represents a substituent selected from the group consisting of the following (a) to (e) (wherein Y represents an integer between 5 and 17):
   (a) -CH₂(CH₂)_{Y}CH₃
   (b) -CH(OH)(CH₂)_{Y}CH₃
   (c) -CH(OH)(CH₂)_{Y}CH(CH₃)₂
   (d) -CH=CH(CH₂)_{Y}CH₃
   (e) -CH(OH)(CH₂)_{Y}CH(CH₃)CH₂CH₃, and
R³ to R⁹ represent substituents as defined In any one of the following i) to v):
   i) when R³, R⁶ and R⁸ represent H,
   R⁴ represents H, OH, NH₂, NHCOCH₃, or a substituent selected from the group consisting of the following groups (A) to (D): 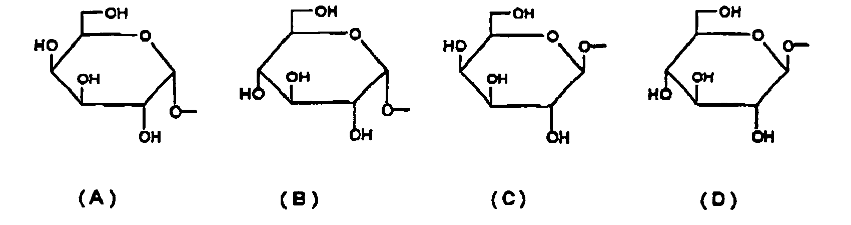
   R⁵ represents OH or a substituent selected from the group consisting of the following groups (E) and (F): 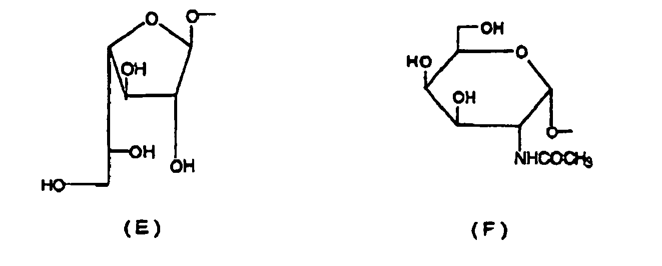
   R⁷ represents OH or a substituent selected from the group consisting of the following groups (A) to (D): 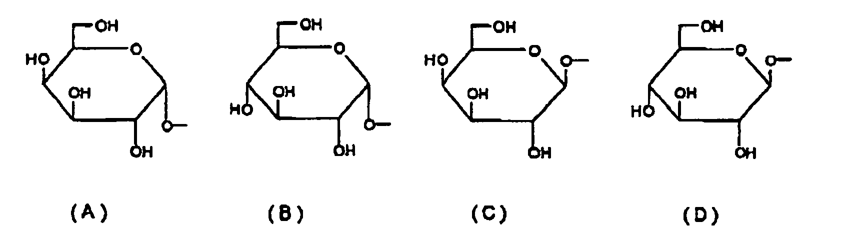
   R⁹ represents H, CH₃, CH₂OH or a substituent selected from the group consisting of the following groups (A') to (D'): 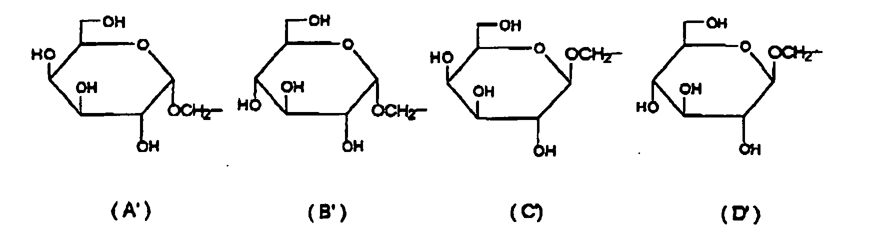
   ii) when R³ , R⁶ and R⁷ represent H,
   R⁴ represents H, OH, NH₂, NHCOCH₃, or a substituent selected from the group consisting of the following groups (A) to (D): 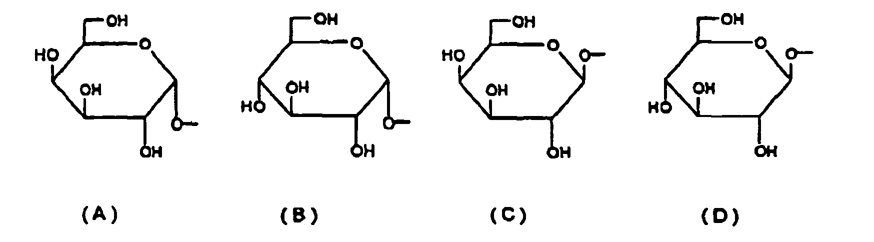
   R⁵ represents OH or a substituent selected from the group consisting of groups (E) and (F): 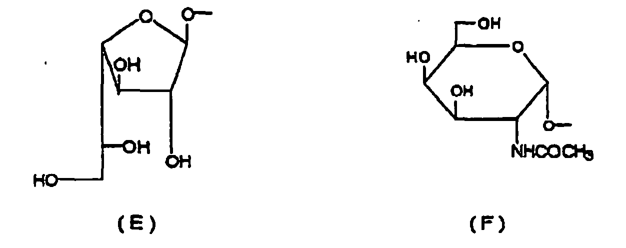
   R⁸ represents OH or a substituent selected from the group consisting of the following groups (A) to (D): 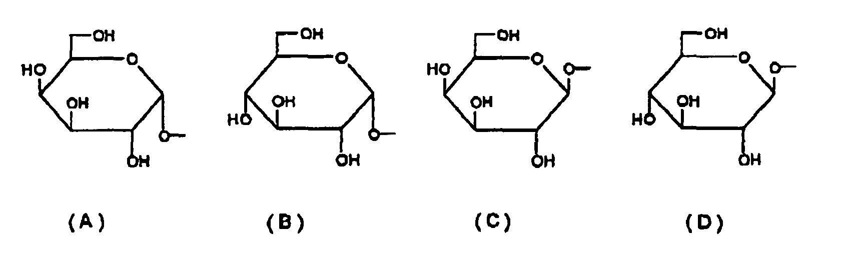
   R⁹ represents H, CH₃, CH₂OH or a substituent selected from the group consisting of the following groups (A') to (D'):

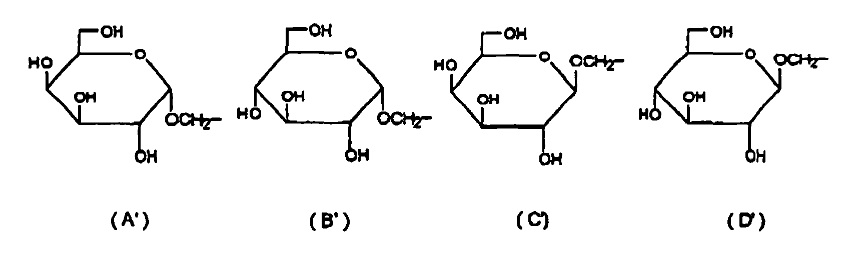

In the compounds of formula (I), X in the ceramide moiety preferably represents an integer between 11 and 25. Y in R² preferably represents an integer between 9 and 17, more preferably between 11 and 15.

Preferable combinations for X and R² in the ceramide moiety of formula (I) are compounds in which X is an integer between 21 and 25 and R² is substituent (b) (wherein Y is an integer between 11 and 15), and compounds in which X is an integer between 9 and 13 and R² is the substituent (a) (wherein Y is an integer between 11 and 15).

Preferable combinations for R³ to R⁹ in the sugar moiety of formula (I) are compounds in which R³ and R⁶ are H, R⁴ is OH or any substituent of groups (A) to (D), R⁵ is OH or any substituent of group (E) or (F), R⁷ and R⁸ are each H or OH (but R⁷ and R⁸ are different from one another), and R⁹ is CH₂OH, CH₃, H or any substituent of groups (A') to (D').

More preferable combinations include compounds in which R³ and R⁶ are H, R⁴ and R⁵ are OH, R⁷ and R⁸ are each H or OH (but R⁷ and R⁸ are different from one another), and R⁹ is CH₂OH or any substituent of groups (A') to (D'), and compounds in which R³, R⁶ and R⁸ are H, R⁴, R⁵ and R⁷ are OH, and R⁹ is CH₂OH.

Preferable examples of compounds of formula (I) include compounds in which X is an integer between 21 and 25, R² is substituent (b) (wherein Y is an integer between 11 and 15), R³ and R⁶ are H, R⁴ is OH or a group selected from the group consisting of groups (A) to (D), R⁵ is OH or a group selected from the group consisting of groups (E) and (F), R⁷ and R⁸ are each H or OH (but both R⁷ and R⁸ are not the same substituent), and R⁹ is CH₂OH or a group selected from the group consisting of groups (A') to (D').

Another preferable examples of compounds of formula (I) include compounds in which X is an integer between 9 and 13, R² is substituent (a) (wherein Y is an integer between 11 and 15), R³ and R⁶ are H, R⁴ and R⁵ are OH, R⁷ and R⁸ are each H or OH (but both R⁷ and R⁸ are not the same substituent), and R⁹ is H, CH₃ or CH₂OH.

Another preferable examples of compounds of formula (I) include compounds in which X is an integer between 21 and 25, R² is substituent (b) (wherein Y is an integer between 11 and 15), R³ and R⁶ are H, R⁴ and R⁵ are OH, R⁷ and R⁸ are each H or OH (but both R⁷ and R⁸ are not the same substituent), and R⁹ is CH₂OH or a group selected from the group consisting of groups (A') to (D').

Another preferable examples of compounds of formula (I) include compounds in which X is an integer between 21 and 25, R² is substituent (b) (wherein Y is an integer between 11 and 15), R³, R⁶ and R⁸ are H, R⁴, R⁵ and R⁷ are OH, and R⁹ is CH₂OH.

More preferable examples of compounds of formula (I) include:
(2S,3S,4R)-1-(α-D-galactopyranosyloxy)-2-hexacosanoylami no-3,4-octadecanediol (KRN 7000),
(2S,3R)-1-(α-D-galactopyranosyloxy)-2-tetradecanoylamino-3-octadecanol (AGL-517),
(2S,3R)-1-(α-D-glucopyranosyloxy)-2-tetradecanoylamino-3-octadecanol (AGL-563),
(2S,3R)-1-(6'-deoxy-α-D-galactopyranosyloxy)-2-tetradecan oylamino-3-octadecanol (AGL-571),
(2S,3R)-1-(β-L-arabinopyranosyloxy)-2-tetradecanoytamino-3-octadecanol (AGL-577),
O-α-D-galactopyranosyl-(1-6)-O-α-D-galactopyranosyl-(1-1)-(2S,35,4R)-2-amino-N-hexacosanoyl-1,3,4-octadecanetriol (AGL-586),
O-α-D-galactopyranosyl-(1-6)-O-α-D-glucopyranosyl-(1-1)-( 2S,3S,4R)-2-amino-N-hexacosanoyl-1,3,4-octadecanetriol (AGL-584),
O-α-D-galactopyranosyl-(1-2)-O-α-D-galactopyranosyl-(1-1)-(2S,3S,4R)-2-amino-N-[(R)-2-hydroxytetracosanoyl]-1,3,4-octadec anetriol (S1140B-9),
O-β-D-galactofuranosyl-(1-3)-O-α-D-gatactopyranosyl-(1-1)-(2S,3S,4R)-2-amino-N-[(R)-2-hydroxytetracosanoyl]-1,3,4-octadec anetriol (719-7), and
O-(N-acetyl-2-amino-2-deoxy-α-D-galactopyranosyl-(1-3)-O-[α-D-glucopyranosyl-(1-2)]-O-α-D-galactopyranosyl-(1-1)-(2S,3S, 4R)-2-amino-N-[(R)-2-hydroxytetracosanoyl]-1,3,4-octadecanetriol (STL-8).

A particularly preferable compound used as an active ingredient in therapeutic agents according to the present invention is (25, 3S, 4R)-1-(α-D-galactopyranosyloxy)-2-hexacosanoylamino -3,4-octadecanediol (KRN 7000).

The compounds of formula (I) may be in the form of pharmaceutically acceptable nontoxic salts thereof. Salts of compounds of formula (I) include acid added salts, such as salts with inorganic acids (e.g., hydrochloric acid, sulfuric acid, nitric acid and phosphoric acid) or with organic acids (e.g., acetic add, propionic add, maleic acid, oleic acid, palmitic acid, citric add, succinic acid, tartaric add, fumaric acid, glutamic acid, pantothenic acid, laurylsulfonic acid, methanesulfonic acid and phthalic add). The compounds of formula (I) may also be in the form of solvates thereof (e.g., hydrates).

The compounds of formula (I) can be produced by any purposive method to synthesize α-glycosylceramides. First, a ceramide moiety is synthesized using D-lyxose as a starting material, then a sugar is introduced into this ceramide to prepare compounds of formula (I). A general method to synthesize such α-glycosylceramides can be found, for example, in WO93/5055, WO94/2168, WO/9020 and WO94/24142. The compounds of formula (I) can also be isolated from natural products (e.g., biological organisms) and purified by column chromatography or the like.

The antigen fragment presented on DC is an antigen fragment that is presented against T cell in the aimed T cell-dependent immune response specific for the antigen. Such an antigen fragment can be appropriately selected according to the aimed immune response by those skilled in the art. For instance, in order to suppress immune response involved in autoimmune disease, the antigen fragment used can be a fragment of antigen present in tissues or organs related to the disease. In order to suppress immune response involved in graft rejection or graft versus host disease, the antigen fragment used can be optionally a fragment of antigen derived from donor or recipient. In order to suppress immune response involved in allergic disease, the antigen fragment used can be a fragment of antigen causing the disease or the antigen which has been modified by exogenous substances and thus obtained novel antigenicity.

DC used for the present invention is preferably, but not limited to, a dendritic cell that is derived from an animal to be treated. The animal is preferably a mammal, more preferably a human being. The human DC is preferably a monocyte-derived DC (Bwatrice Thurner, Gerold Schuler et al., J. Exp. Med. 1999, 190(11): 1669-1678; Axel Heiser, Eli Gilboa et al., J. Clin. Invest. 2002, 109(3): 409-417), a human peripheral blood DC (Small Ej., L. Clin. Oncol. 2000, 18(23): 3894-3903), or a human CD34 positive cell-derived DC (Caux C, Jacques Banchereau et al., Blood 1997, 90(4): 1458-1470).

In the DC used for the present invention, the antigen fragment and the α-glycosylceramide derivative are preferably presented at the MHC molecule and the CD1d molecule, respectively.

The DC is produced ex vivo. A method of producing the DC is described, comprising a step of culturing dendritic cell in the presence of a-glycosylceramide derivative. In another aspect, the use of α-gylcosylceramide derivative for the manufacture of DC is described. The amount of α-glycosylceramide derivative used can be appropriately determined by those skilled in the art and is preferably, but not limited to, in the concentration of 1-1000 ng/ml, more preferably 10-100 ng/ml. The culturing time period can be appropriately determined by those skilled in the art and is preferably, but not limited to, 1-10 days including the last day of culture.

In order to suppress immune response in graft rejection or graft versus host disease, the donor- or recipient-derived dendritic cells which have already presented required antigen can be used to produce the DC for administration to the recipient or donor. Similarly, in order to suppress immune response in the other diseases, the patient-derived dendritic cells which have already presented required antigen can be used to produce the DC for administration to the patient. In these cases, only the culturing step in the presence of α-glycosylceramide derivative may be practiced in the manufacturing method.

If necessary, the manufacturing method may further comprise a step of culturing the dendritic cells in the presence of the antigen or the antigen fragment. When the DC is produced using dendritic cells which do not present required antigen, this step is required. The amount of antigen or fragment used can be appropriately determined by those skilled in the art and is preferably, but not limited to, in the range of 1 µg-10 mg/ml, more preferably 10 µg-2 mg/ml, most preferably 10-100 µg/ml. The culturing time period can be appropriately determined by those skilled in the art and is preferably, but not limited to, 1-10 days including the last day of culture. The antigen fragment may also be presented on the dendritic cells by using DNA or RNA coding for antigen or antigen fragment or modification derivatives thereof in place of the antigen or antigen fragment, and such presentation can be carried out by the method well-known to those skilled in the art.

The other culturing conditions in each of the two steps described above may be those used for an ordinary cell culture. That is to say, conditions including the amounts of cell or cell fraction added to culture medium, culturing temperature, CO₂ concentration and culturing time period can be appropriately set by those skilled in the art. The culturing temperature is preferably about 37°C. The CO₂ concentration may be regulated as desired, and can be preferably about 5%. Furthermore, cytokines including GM-CSF (e.g. 10 ng/ml) or FCS (e.g. 1%) may be added to culture medium, if desired. When both of the two steps are conducted, either step may be conducted first or the both steps may be conducted simultaneously.

The specific antigen-presenting DC and the a-glycosylceramide derivative-presenting DC used in the present invention can also be produced by the same method as described above. That is to say, the specific antigen-presenting DC can be produced, for example, by carrying out the above described step of culturing the dendritic cell in the presence of antigen or antigen fragment. The α-glycosylceramide derivative-presenting DC can be produced, for example, by carrying out the above described step of culturing the dendritic cell in the presence of a-glycosylceramide derivative.

The DC can be used in the cell therapy for the purpose of the treatment of disease caused by T cell-dependent immune response. Thus, the present invention may be useful for a method for treatment of disease induced by T cell-dependent immune response, comprising a step of administering a therapeutically effective amount of the DC to a subject. The subject is preferably mammals, including human or non-human mammals. The T cell is preferably CD4 positive T cell or CD8 positive T cell.

Furthermore, the cell mixture and the combination can be used in cell therapy for the purpose of treatment of disease induced by T cell-dependent immune response. Thus, the present invention may be used in a method for treatment of disease induced by T cell-dependent immune response, comprising a step of administering a therapeutically effective amount of the cell mixture to a subject. Furthermore, the present invention may be useful for a method for treatment of disease induced by T cell-dependent immune response, comprising a step of administering dendritic cells presenting at least antigen fragment of antigen which is presented against T cell in said immune response, and dendritic cells presenting at least α-glycosylceramide derivative, separately or simultaneously, to a subject in a therapeutically effective amount. The subject is preferably mammals, including human or non-human mammals. The T cell is preferably CD4 positive T cell or CD8 positive T cell. In one embodiment, the therapeutic method comprises a step of administering the specific antigen-presenting DC and the α-glycosylceramide derivative-presenting DC separately or simultaneously. In another embodiment, the therapeutic method comprises a step of administering the specific antigen-presenting DC and/or the α-glycosylceramide derivative-presenting DC, and the DC presenting both, separately or simultaneously. In the preferred embodiment of the present invention, these DCs are administered simultaneously.

The diseases induced by T cell-dependent immune response are preferably selected from the group consisting of autoimmune diseases, graft rejection, graft versus host disease, and allergic diseases. The autoimmune diseases include, for example, multiple screlosis, rheumatoid arthritis, type I diabetes, uveitis, autoimmune myocarditis, myasthenia gravis, systemic lupus erythematosus, autoimmune hemolytic anemia, systemic scleroderma, ulcerative colitis, Crohn's disease, Sjögren's syndrome, autoimmune hepatic disease (e.g., primary biliary cirrhosis), psoriasis, idiopathic thrombocytopenic purpura, Goodpasture's syndrome (e.g., glomerulonephritis), pernicious anemia, Hashimoto's disease, vitiligo vulgaris, Behet's disease, autoimmune gastritis, pemphigus, Guillain-Barré syndrome, HTLV-1 associated myelopathy, and the like. The allergic diseases include, for example, contact hypersensitivity, allergic rhinitis, food allergy, asthma, and the like. The delayed type hypersensitivity reaction (DTH reaction) is typical Th1 response, which is regarded as the basic reaction of chronic inflammation in organ-specific autoimmune disease. The organ-specific autoimmune disease in which Th1 immune response is involved includes, principally, multiple sclerosis, rheumatoid arthritis, type I diabetes, uveltis, autoimmune myocarditis, Crohn's disease. The allergic diseases in which Th1 immune response is involved include contact hypersensitivity and the like.

The DC as described herein can be administered in a therapeutically effective amount through a dosage route as usually used in the field of cell therapy. The dosage route is preferably, but not limited to, parenteral administration, more preferably intravenous administration. The therapeutically effective amount is appropriately determined in view of the conditions of a subject, such as the age, weight, sexuality, difference of disease, seriousness of symptom, and the like, and is preferably, but not limited to, 5x10⁵-10⁹, more preferably 0.5-60x10⁶, most preferably 2-20x10⁶ calculated on DC fraction. In addition, the timing of administration may be any of before and after immune response, and before and after the development of the disease, and preferably, but not limited to, at remission. Particularly, for the treatment of graft rejection and graft versus host disease involved in the implantation of organ or tissue, administration prior to the procedure expected to induce the development is preferred.

The dosage route and dosage timing of DC contained in the cell mixture or the combination are the same as described above with regard to the DC alone. The therapeutically effective amount is not specifically limited and appropriately determined in view of the conditions of a subject, such as the age, weight, sexuality, difference of disease, seriousness of symptom, and the like. The therapeutically effective amount is preferably determined such that the total amount of the antigen fragment and α-glycosylceramide derivative presented on DCs to be administered is equal to the amount described above for the DC presenting both. For instance, when the combination of specific antigen-presenting DC and α-glycosylceramide derivative-presenting DC is administered, the dosage amount of each DC is preferably 5x10⁵-10⁹, more preferably 0.5-60x10⁶, most preferably 2-20x10⁶ calculated on DC fraction. Furthermore, when the DC is also administered in combination, the dosage amount of specific antigen presenting-DC and α-glycosylceramide derivative-presenting DC is preferably the amount obtained by subtracting that of the DC presenting both from each dosage amount described above. In addition, the present invention provides a use of the DC as described above for the manufacture of a medicament for the treatment of disease described above.

Furthermore, the present invention provides a use of the cell mixture as described above for the manufacture of a medicament for the treatment of disease described above.

Moreover, the present invention provides a use of the combination as described above for the manufacture of a medicament for the treatment of disease described above, i.e., a use of a combination of dendritic cells for the manufacture of a medicament for the treatment of disease induced by T cell-dependent immune response, wherein said combination consists of dendritic cells presenting at least antigen fragment of an antigen which is presented against T cell in said immune response, and dendritic cells presenting at least α-glycosylceramide derivative.

Furthermore, a pharmaceutical composition is described for the treatment of disease described above, comprising the DC as described above

Furthermore, a pharmaceutical composition is described for the treatment of disease described above, comprising the cell mixture as described above.

These pharmaceutical compositions can be appropriately produced by those skilled in the art, depending on the dosage route and dosage amount described above, or according to the well-known techniques in cell therapy.

### EXAMPLES

The present invention is now explained in detail by the following examples, but the present invention is not limited to these examples.

### Example 1: Effect of immune regulatory DC on autoimmune disease model

### 1-1: Induction and separation of DC from bone marrow cell.

Femora were excised from C57BL/6 mice at 6-8 weeks of age, and Hanks' solution (Gibco BRL) was injected into the femora through a syringe provided with a 25G needle to wash out bone marrow cells. The cells were hemolized with ammonium chloride buffer (SIGMA) to remove erythrocyte, and then inoculated on a plate coated with human γ globulin (SIGMA). The plated cells were incubated under the ordinary culturing condition to remove Fc receptor-expressing cells and to concentrate precursor cells of DC. The cells were suspended in an RPMI1640 culture medium supplemented with GM-CSF (KIRIN) (10 ng/ml), FCS (Hyclone) (10%), HEPES buffer (SIGMA) (10 mM) and 2-mercaptoethanol (Gibco BRL) (50 µM), and cultured on a 24 well plate under a condition of 5.0% of CO₂ at 37°C. After 2 and 4 days of the initiation of culturing, the following procedure for medium-exchange and cell-concentration was carried out. The medium-exchange was carried out by mildly suspending the cells with a Pasteur pipette, removing 3/4 of supernatant after the sinking of cell cluster, and adding the fresh medium described above which contains cytokines. After 6 days from the start of culturing, the procedure until the removal of supernatant was conducted in the same manner, and all of the remaining cells were recovered and used as DC fraction. After magnetic beads-labeled anti-CD11c antibody (Miltenyi Biotec GmbH) was added to the DC fraction, DC was separated by magnetic cell separation system (Miltenyi Biotec GmbH).

### 1-2: Preparation of modified DC

DC separated in the section 1-1 was cultured in RPMI1640 medium (containing 10 ng/ml of GM-CSF) which contains either or both of peptide corresponding to the 35-55 residues of myelin oligodendrocyte glycoprotein (MOG; amino acid sequence: MEVGWYRSPFSRWHLYRNGK) (10 µM) and KRN7000 (100ng/ml) for 2 days under the condition of 5.0% CO₂ at 37°C (a step of affording MOG and/or KRN7000) to obtain a modified DC. In this connection, MOG is an antigen used later in the immunization which induces experimental allergic encephalitis (EAE).

### 1-3: Analysis of character of DC obtained

Cells obtained in the sections 1-1 and 1-2 were confirmed to be DC by staining with the following antibodies and flow cytometry. Specifically, CD11c which is a marker specific to mouse DC was stained for analysis by the following method. Anti-CD11c antibody (BD pharmingen) or isotype control thereof, i.e. hamster IgG (BD pharmingen), was added to the cells suspended in a washing buffer (PBS containing 2% FCS/0.02% sodium azide) at a concentration of 5 µg/ml for the reaction at 4°C for 30 minutes, washed and analyzed with a fluorescence activated cell sorter (FACS). It has been confirmed for either of the above described cells that CD11c positive cell are in a proportion of 97% or more and the most is DC. The proportion was not changed even by the addition of MOG or KRN7000. In addition, it has been revealed by the fluorescence activated cell sorter (FACS) (BD Biosciences) using anti-MHC class II (I-A) antibody and anti-CD86 antibody that the most of the cells is MHC class II (I-A) positive and CD86 positive.

### 1-4: Effect on EAE

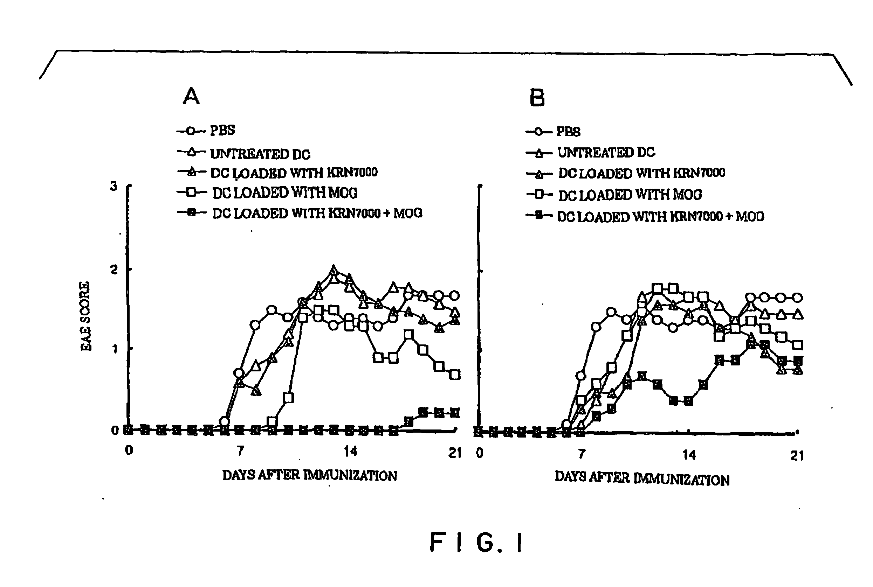

DC obtained in the sections 1-1 and 1-2 was administered before immunization with antigen for inducing EAE (three times of at 7, 5 and 3 days before immunization; Fig. 1A), or at one day after immunization (Fig. 1B). A group treated with phosphate buffered saline (PBS), a group treated with untreated DC, a group treated with DC which was loaded with KRN7000, a group treated with DC which was loaded with MOG, and a group treated with DC which was loaded with both of KRN7000 and MOG were provided as experimental groups. Each of the groups treated with various DCs were divided into two sub-groups for administration at the above described two periods. To mice in the group treated with PBS, PBS was administered In place of DC on both days of DC administration before and after immunization with antigen.

EAE was induced by administering 200 µL of an antigen (MOG) emulsion intradermally to a proximal portion of tail of C57BL/6 mice at 9 weeks of age, and administering intraperitoneally 400 ng of pertussis toxin (Pertassis toxin: Seikagaku Corporation) on the next day. Each of the groups consists of 9-10 mice. For preparation of the antigen emulsion, physiological saline containing 200 µg/100 µL of MOG and 600 µg/100 µL of Mycobacterium tuberculosis H37RA (Difco) was emulsified with an equal volume of Freund's complete adjuvant (Difco).

Body weight and EAE symptoms were observed for three weeks starting from the day of antigen immunization as day 0. EAE symptoms were evaluated by scoring in six ranks of 0 = no symptom, 1 = lowering of autokinesis of tail, 2 = failure of righting reflex, 3 = medium paralysis of posterior limb, 4 = complete paralysis of posterior limb, 5 = quadriplegia, and 6 = death.

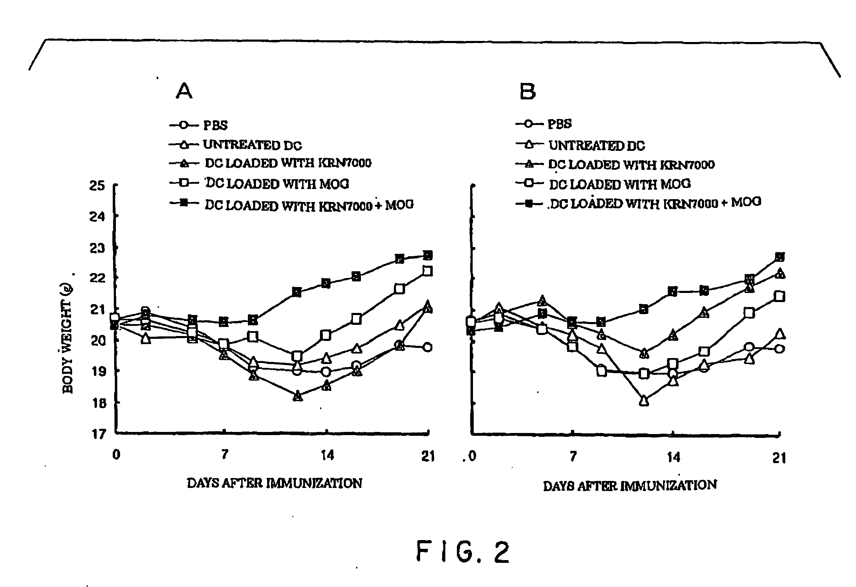

The result is shown in Fig. 1 and Fig. 2, and Table 1. Table 1 shows the values of various parameters (average cumulative EAE score (AUC), average development day, average maximum score, and percentage of development) related to symptom of EAE.

**Table 1: Effect of Suppressing EAE by Immune Regulatory DC**

| Experimental group (administered agent) | Day of administration | n | Average cumulative score (AUC) | Average of development day | Average max score | Percentage of development |
|---|---|---|---|---|---|---|
| PBS | -7, -5, -3, 1 | 10 | 20.8±7.0 | 9.0±3.61 | 2.0±0.0 | 100 |
| Untreated DC | -7, -5, -3 | 10 | 21.4±4.6 | 9.3±2.2 | 2.0±0.0 | 100 |
| DC loaded with | -7, -5, -3 | 10 | 20.1±6.0 | 9.1±2.1 | 2.1±0.7 | 100 |
| KRN7000 | | | | | | |
| DC loaded with | -7, -5, -3 | 10 | 12.7± 8.3* | 10.4±0.7 | 1.6±0.7 | 90 |
| MOG | | | | | | |
| DC loaded with | -7, -5, -3 | 9 | 0.7±2.0** | 18.0 | 0.2±0.7** | 11** |
| KRN7000 + MOG | | | | | | |
| Untreated DC | 1 | 10 | 19.4±7.9. | 9.3±1.3 | 1.8±0.4 | 100 |
| DC loaded with | 1 | 10 | 16.7±9.4 | 9.4±1.8 | 1.6±0.8 | 80 |
| KRN7000 | | | | | | |
| DC loaded with | 1 | 10 | 18.6±7.8 | 8.9±1.5 | 1.8±0.6 | 90 |
| MOG | | | | | | |
| DC loaded with | 1 | 10 | 9.2±8.9** | 12.1±3.8 | 1.3±0.9* | 70 |
| KRN7000 + MOG | | | | | | |

| | | | | | | |
|---|---|---|---|---|---|---|
| Day of administration was counted from Day 0 on which EAE is induced. Only PBS was administered to mice in the group for PBS administration on Day of administration. The significant difference against PBS-administered group were determined by Student's t-test, except for Percentage of development for which χ² distribution test was used (*p<0.05, **p<0.01). | | | | | | |

In the group treated with DC which was loaded with both of KRN7000 and MOG, the suppression of the development or symptom of EAE was observed when the DC was administered at either timing, and the suppressive effect was more remarkable in the group treated with DC three times prior to the induction of EAE (Fig. 1 and Table 1). In addition, the EAE-suppressing effect in the group treated with DC which was loaded with both of KRN7000 and MOG involved the suppression of body weight decrease associated with EAE (Fig. 2), and not only the average cumulative EAE score (AUC: area under the curve) but also average maximum score, and frequency of development (only the group treated with the DC three times prior to the induction of EAE) were significantly different from the group treated with PBS (Table 1). In this connection, the group treated with DC which was loaded with MOG three times before EAE induction also showed significant suppression of average cumulative EAE score, but the degree of suppression was inferior to the group treated with DC which was loaded with KRN7000 and MOG simultaneously (Table 1).

### Example 2: Suppressive effect of immune-regulatory DC on inducing cytotoxic CD8 positive T cell (CTL)

### 2-1: Preparation of DC treated with OVA peptide as antigen

DC obtained in the section 1-1 was further cultured in RPMI1640 medium containing GM-CSF (10 ng/ml) at 37°C under the condition of 5% CO₂ for two days. During culturing for two days, DCs were divided into two groups: treated and not treated with KRN7000. After replacing the medium with RPMI1640 medium containing 1% FCS and adding with the synthesized OVA peptide (SIINFEKEL) (Quiagen), the mature DC was cultured under the same condition for four hours. The mature DC collected after four hours was washed with PBS and adjusted the concentration to 5x10⁵ cells/ml.

### 2-2: Effect of KRN7000 on the cancer therapeutic effect by the DC treated with OVA peptide as an antigen

DC obtained in the section 2-1 was suspended in PBS to obtain the concentration of 1x10⁵ cells/200 µl per mouse and injected intravenously to mice. Further, seven days later, E.G7 cells (EL-4 cells transfected with OVA gene) or EL-4 cells as a control were injected subcutaneously in the right flank of the mice, and the volume of tumor was measured with the passage of time. The volume of tumor was calculated as length x width x height of tumor/2.

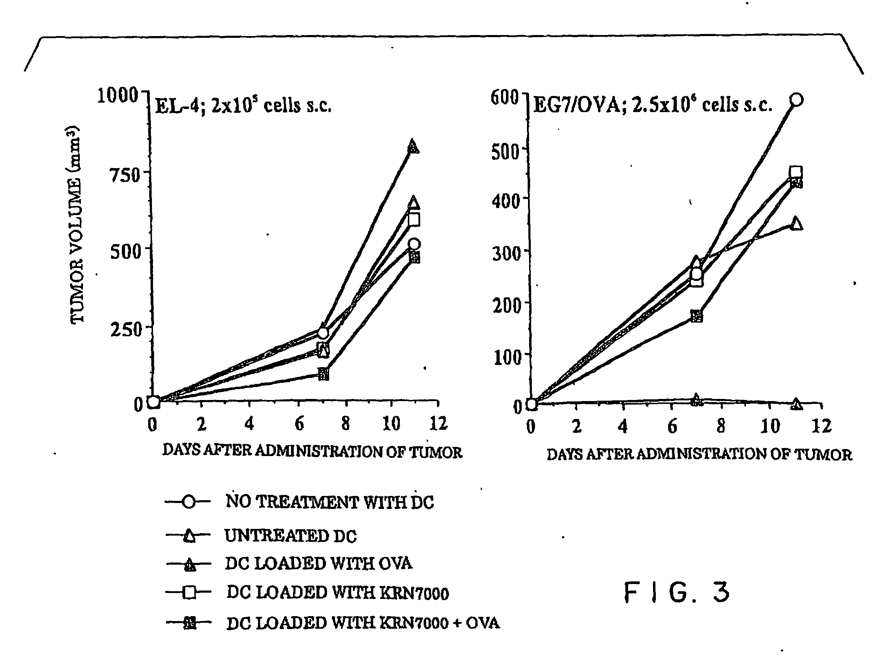

As a result, proliferation of tumor was considerably inhibited In the group of mice treated with DC having only OVA peptide loaded with thereto, whereas in the group treated with DC having KRN7000 and OVA peptide loaded with thereto, proliferation of tumor equivalent to that in the untreated group was observed, and thus remarkable inhibition of tumor proliferation-suppressing effect was observed (Fig. 3).

### 2-3: Effect on the induction of peptide specific IFNγ production with OVA peptide as an antigen

Splenic lymphocytes were collected from mice treated with DC obtained in the section 2-1 with the passage of time (3, 5, 7 and 9 days later). Spleen was swollen in RPMI1640 medium, and was crushed with slide glass. The obtained cells were hemolyzed with buffer containing NH₄Cl to give splenic lymphocytes, which were suspended into Clicks' medium (Irvine science) and cultured under the condition of adding or not adding OVA peptide for three days to measure the production of IFNγ in supernatant by ELISA.

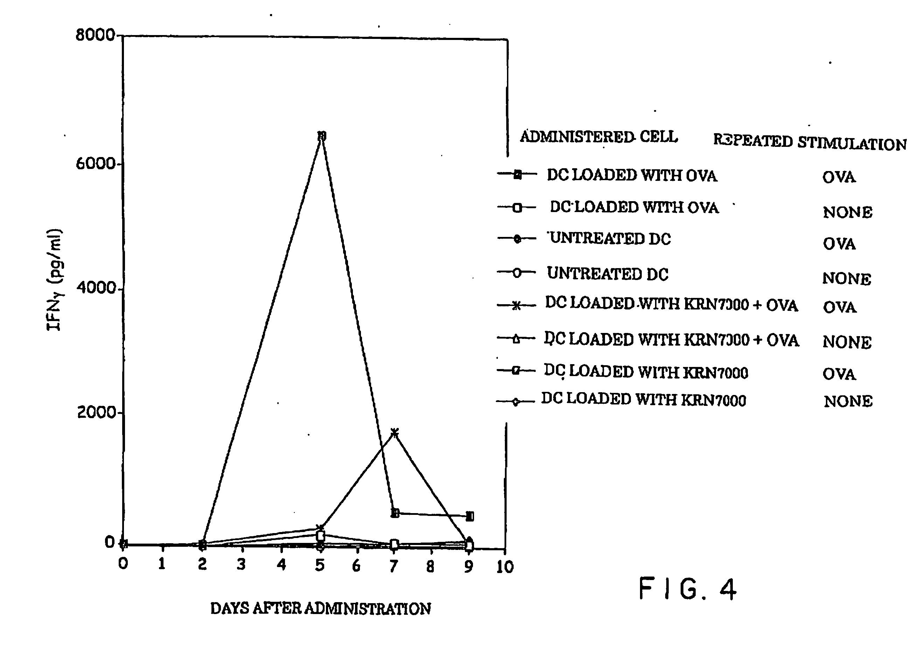

As a result, OVA peptide-specific IFNγ production with a peak at five days after administration was observed in mice treated with DC having only OVA peptide loaded with thereto, whereas OVA peptide-specific IFNγ production was inhibited in mice treated with DC having KRN7000 and OVA peptide simultaneously loaded with thereto (Fig. 4).

### Example 3: Effect on type II collagen induced arthritis in mice

Induction and separation of mouse DC were conducted in the same manner as in Example 1-1.

The separated DC was cultured in RPMI1640 medium (containing 10 ng/ml of GM-CSF) which contains either or both of bovine type II collagen (K-41, COLLAGEN GIJUTSU KENSHUKAI) (1 mg/ml) previously boiled for 10 minutes at 95-100 °C and KRN7000 (100 ng/ml) for 2 days under the condition of 5.0% CO₂ at 37°C (a step of affording K-41 and/or KRN7000) to obtain a modified DC. In this connection, bovine type II collagen is an antigen used later in the immunization which induces type II collagen induced arthritis (CIA).

In experiments, ten male DBA/1JNCrj mice (11 weeks of age) (Charles River Japan, Inc.) were used per group. First, an emulsion was prepared from the mixed solution of 0.3% bovine type II collagen containing solution (5 ml) (K-41, COLLAGEN GIJUTSU KENSHUKAI), Mycobacterium tuberculosis H37Ra (15 mg) (Difco), physiological saline solution (2.5 ml) (Otsuka Pharmaceutical Co., Ltd.), and incomplete Freund's adjuvant (7.5 ml) (Difco). The resultant was used as an antigen emulsion. In the next step, type II collagen induced arthritis (CIA) was induced by injecting twice 0.1 ml of the antigen emulsion intradermally to a proximal portion of tail of mice with an interval of three weeks. Administration of DC (1x10⁶ cells/mouse) into caudal vein of mice was carried out one day after the initial injection of the antigen.

Body weight and arthritis symptoms of mice were observed starting from the day of the second antigen injection as day 0. Clinical scoring for each paw was assessed by reference to the following scale: 0= normal, 1= swelling and/or erythema of one toe, 2= swelling and/or erythema of two or more toes, 3= swelling and erythema of the entire paw, 4= complete swelling and erythema of the entire paw and incapacity to bend the ankle. CIA score was expressed as the cumulative value for all paws, with a maximum of 16.

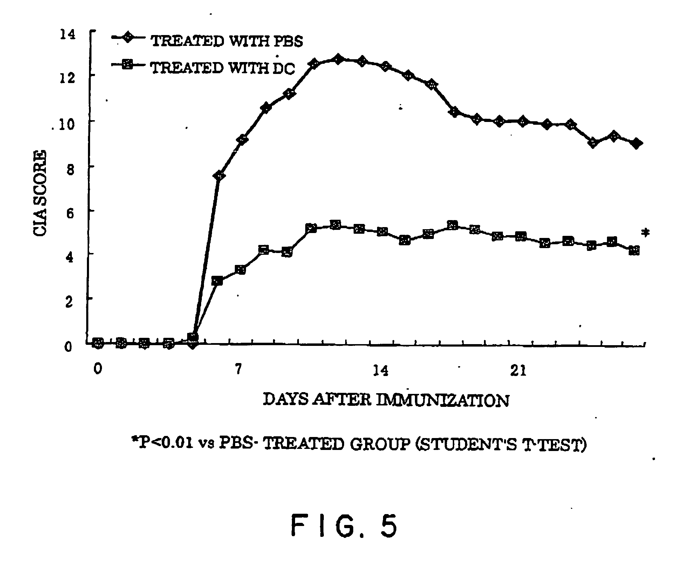

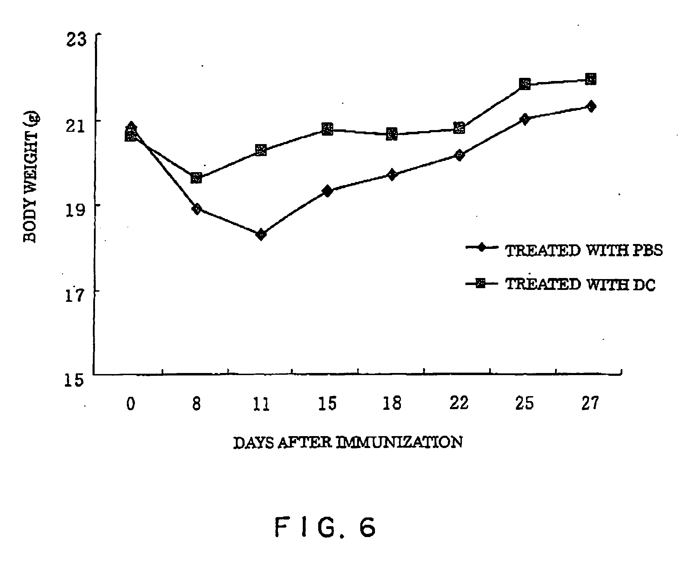

The result is shown in Fig. 5 and Fig. 6. Significant suppression of arthritis symptom (average cumulative CIA score (AUC)) was observed in the group treated with DC which was loaded with both of KRN7000 and type II collagen (Fig. 5). Further, the arthritis suppressing effect in the group treated with DC which was loaded with both of KRN7000 and type II collagen involved the suppression of body weight decrease associated with arthritis (Fig. 6).

### Example 4 : Effect of suppressing the development of experimental autoimmune encephalomyelitis (EAE) by the mixed administration of DC which was loaded with KRN7000 and DC which was loaded with MOG peptide

DC prepared as described in Example 1-2 was administered two days after immunization with antigen for the induction of EAE. As experimental groups, a group treated with phosphate buffered saline (PBS), two groups treated with DC which was loaded with both of KRN7000 and MOG at dosages of 5x10⁵ cells/mouse and 1x10⁶ cells/mouse respectively, and a group treated with the mixture of DC which is loaded with KRN7000 (5x10⁵ cells/mouse) and DC which is loaded with MOG (5x10⁵ cells/mouse) were provided, and intravenous injection was carried out for all of the groups.

EAE was induced by administering 200 µl of an antigen (MOG) emulsion intradermally to a proximal portion of tail of C57BL/6 mice at 9 weeks of age, and administering intraperitoneally 400 ng of pertussis toxin (Pertassis toxin: Seikagaku Corporation) on the next day. Each of the groups consists of 9-10 mice. For preparation of the antigen emulsion, physiological saline containing 200 µg/100 µL of MOG and 600 µg/100 µL of Mycobacterium tuberculosis H37RA (Difco) was emulsified with an equal volume of Freund's complete adjuvant (Difco).

Body weight and EAE symptoms were observed for four weeks starting from the day of antigen immunization as day 0. EAE symptoms were evaluated by scoring in six ranks of 0 = no symptom, 1 = lowering of autokinesis of tail, 2 = failure of righting reflex, 3 = medium paralysis of posterior limb, 4 = complete paralysis of posterior limb, 5 = quadriplegia, and 6 = death.

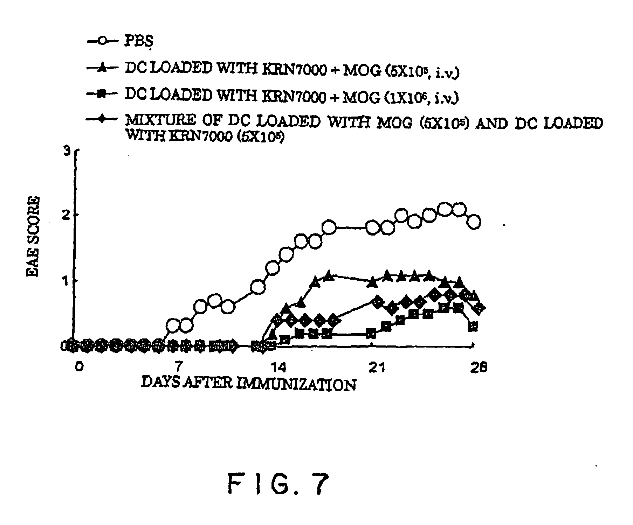

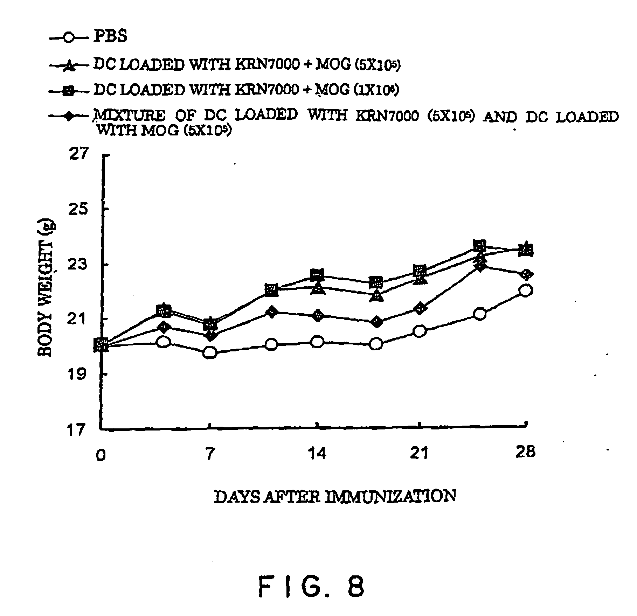

The result is shown in Fig. 7 and Fig. 8, and Table 2. Table 2 shows the values of various parameters (average cumulative EAE score (AUC), average development day, average maximum score, and percentage of development) related to symptom of EAE.

**Table 2: EAE-suppressing effect in respective experimental groups after induction of EAE**

| Experimental group (administered agent) | n | Average cumulative score (AUC) | Average developmen t day | Average maximum score | Percentage of develop ment |
|---|---|---|---|---|---|
| PBS | 10 | 41.6±7.1 | 11.6±3.9 | 2.1±0.3 | 100 |
| DC loaded with KRN7000 + MOG (5x10⁵, i.v.) | 10 | 24.5±17.4* | 15.7±1.6* | 1.4±1.1 | 70 |
| DC loaded with KRN7000 + MOG (1x10⁶, i.v.) | 10 | 13.0±16.8** | 21.5±4.7** | 0.7±0.9** | 40^{#} |
| The mixture of DC loaded with MOG (5x10⁵) and DC loaded with KRN7000 (5x10⁵) | 10 | 15.5±20.2** | 17.5±4.0* | 0.8±1.0** | 40^{#} |

| | | | | | |
|---|---|---|---|---|---|
| * p<0.05 vs group for PBS administration (Student's t test) ** p<0.01 vs group for PBS administration (Student's t test) # p<0.05 vs group for PBS administration (χ² distribution test) | | | | | |

In the group treated with DC which was loaded with both of KRN7000 and MOG, significant suppression of the development of EAE depending on the number of cells administered was observed, and also in group treated with the mixture of DC which was loaded with KRN7000 and DC which was loaded with MOG, significant suppressive effect of the development and symptom of EAE was observed (Fig. 7). These EAE-suppressing effects were observed significantly not only in the average cumulative EAE score (AUC: area under the curve) but also in the average development day, the average maximum score, and the percentage of development, in comparison with the PBS group (Table 2). Further, the EAE-suppressing effect involves the suppression of body weight decrease associated with EAE (Fig. 8).

### Example 5: Effect of suppressing the development of delayed hypersensitivity (DTH reaction) caused by OVA as an antigen by administering DC which was loaded with both of KRN7000 and OVA antigen

DC separated in the section 1-1 was cultured in RPMI1640 medium (containing 10 ng/ml of GM-CSF) which contains either or both of OVA (2 mg/ml) and KRN7000 (100 ng/ml) for 2 days under the condition of 5.0% CO₂ at 37°C (a step of affording OVA antigen and/or KRN7000) to obtain a modified DC. In this connection, the OVA is an antigen used later in the immunization which induces delayed type hypersensitivity (DTH reaction).

DC which was loaded with KRN7000 or DC which was loaded with both of KRN7000 and OVA antigen (1.5x10⁶ cells/mouse) was intravenously injected into C57BL/6 mice three times of at 7, 5 and 3 days before immunization with OVA. PBS (PBS group) or DC which was loaded with nothing (untreated DC group) was administered to the control group in the same manner. In this experiment, a group non-sensitized with OVA was provided in addition to the above control group. Further, a group treated with Prednisolone (0.2 mg/mouse of Prednisolone acetate (Shionogi & Co., Ltd.) were injected intraperitoneally on 9, 10 and 11 days after immunization) was provided as a positive control group. Five and ten mice were used for the OVA non-sensitized group and the other groups, respectively.

DTH reaction with OVA as an antigen was induced by sensitization through injecting intradermally an emulsion which was prepared by adding 100 µg of ovalbumin (OVA) to complete Freund's adjuvant (CFA) (Sigma) into the back of mice for immunization on the day 0, and administering the solution of 10 µg of OVA in 20 µl of PBS to auricle after 10 days. On the next day, auricles of mice having a diameter of 5 mm with a center of the part in which OVA was injected was collected by punching with a punch for cutaneous biopsy, the weight thereof was measured on an electronic balance, and measurement obtained were used as an index for representing the degree of DTH.

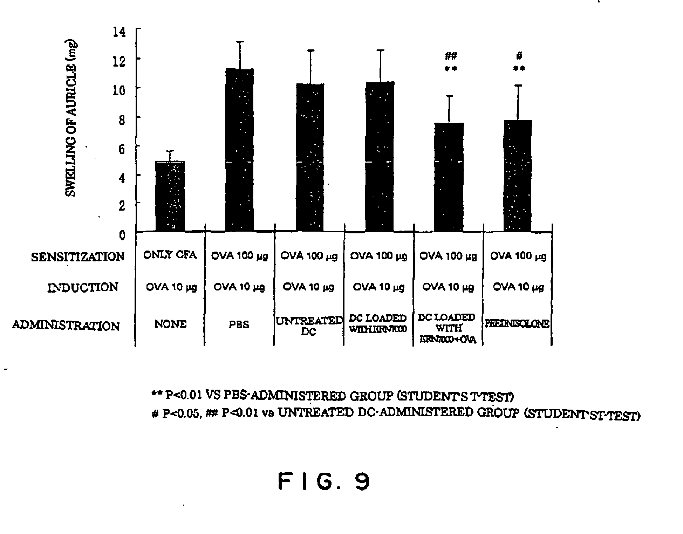

As a result of the experiment described above, significant suppressive effect on the swelling of auricle was observed in the group treated with DC which was loaded with both of KRN7000 and OVA antigen, and the effect was equal to or higher than the suppressive effect of Prednisolone as a positive control (Fig. 9).

## Claims

1. Use of a dendritic cell for the manufacture of a medicament for the treatment of disease selected from the group consisting of autoimmune disease, graft rejection, graft versus host disease, and allergic disease,
wherein said dendritic cell presents antigen fragment of an antigen which is presented against T cell in T cell dependent immune response inducing said disease, and alpha-glycosylceramide derivative.

2. Use as claimed in claim 1 wherein the alpha-glycosylceramide derivative is a compound of formula (I): 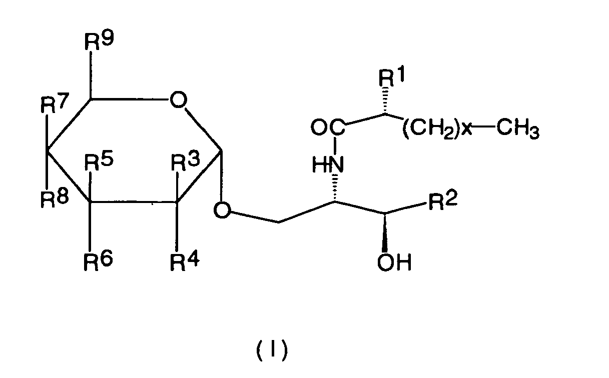 wherein
R¹ represents H or OH,
X represents an integer between 7 and 27,
R² represents a substituent selected from the group consisting of the following (a) to (e) (wherein Y represents an integer between 5 and 17):
(a) -CH₂(CH₂)_{Y}CH₃
(b) -CH(OH)(CH₂)_{Y}CH₃
(c) -CH(OH)(CH₂)_{Y}CH(CH₃)₂
(d) -CH=CH(CH₂)_{Y}CH₃
(e) -CH(OH) (CH₂)_{Y}CH(CH₃)CH₂CH₃, and
R³ to R⁹ represent substituents as defined in the following i) or ii) :
i) when R³, R⁶ and R⁸ represent H,
R⁴ represents H, OH, NH₂, NHCOCH₃, or a substituent selected from the group consisting of the following groups (A) to (D): 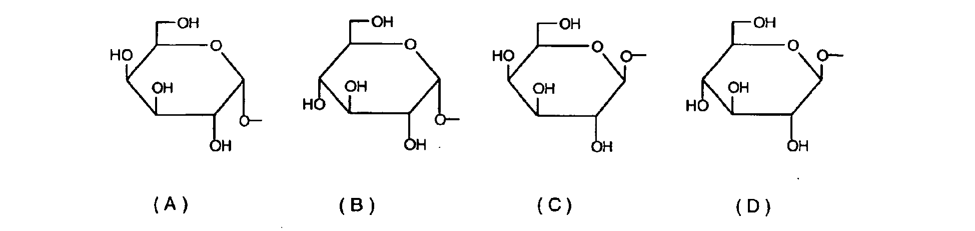
R⁵ represents OH or a substituent selected from the group consisting of the following groups (E) and (F): 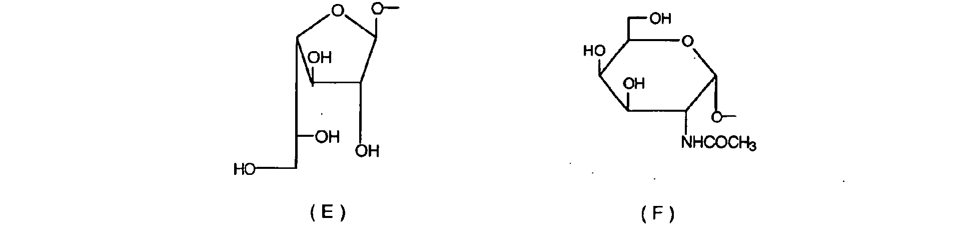
R⁷ represents OH or a substituent selected from the group consisting of the following groups (A) to (D): 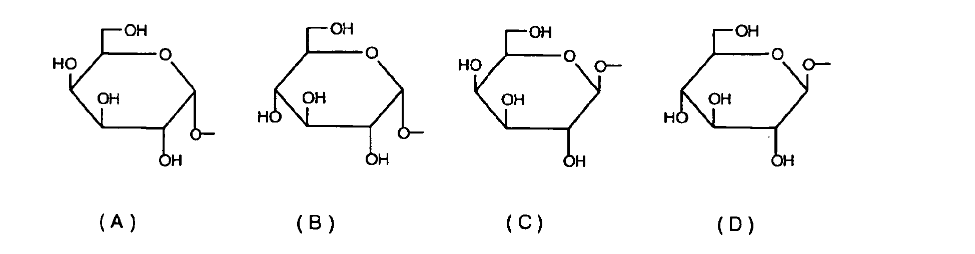
R⁹ represents H, CH₃, CH₂OH or a substituent selected from the group consisting of the following groups (A') to (D'): 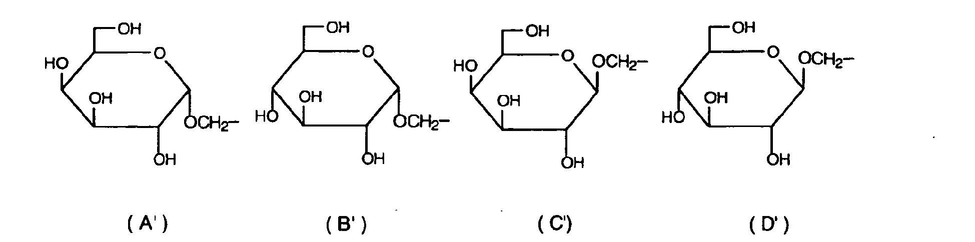
ii) when R³, R⁶ and R⁷ represent H,
R⁴ represents H, OH, NH₂, NHCOCH₃, or a substituent selected from the group consisting of the following groups (A) to (D): 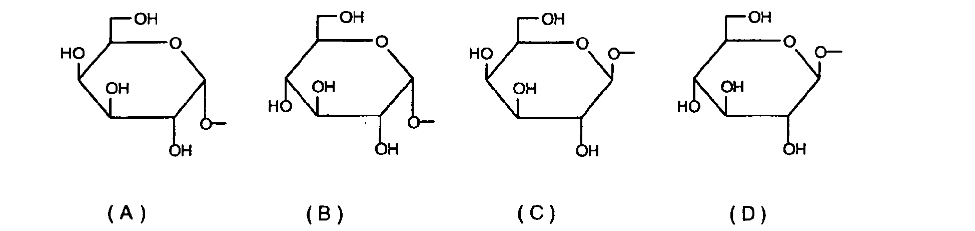
R⁵ represents OH or a substituent selected from the group consisting of the following groups (E) and (F): 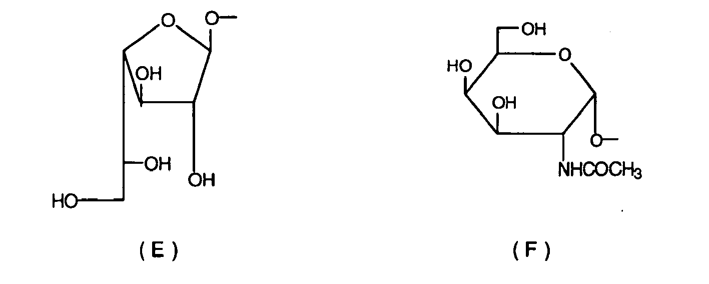
R⁸ represents OH or a substituent selected from the group consisting of the following groups (A) to (D): 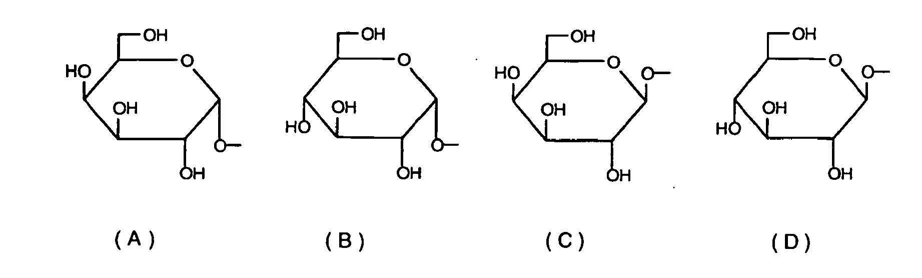
R⁹ represents H, CH₃, CH₂OH or a substituent selected from the group consisting of the following groups (A') to (D'): 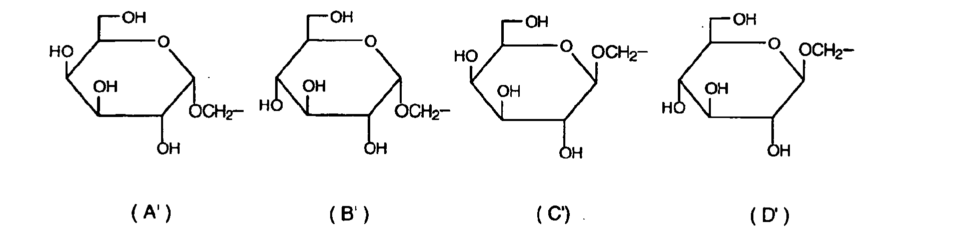
or a salt or a solvate thereof.

3. Use as claimed in claim 2 wherein R³ and R⁶ represent H, R⁴ represents OH or a substituent of any one of groups (A) to (D), R⁵ represents OH or a substituent of group (E) or (F), R⁷ and R⁸ each represent H or OH wherein both R⁷ and R⁸ do not represent the same substituent, and R⁹ represents CH₂OH, CH₃, H or a substituent of any one of groups (A') to (D').

4. Use as claimed in claim 2 wherein X represents an integer between 21 and 25 and R² represents substituent (b) wherein Y represents an integer between 11 and 15.

5. Use as claimed in claim 2 wherein X represents an integer between 9 and 13 and R² represents substituent (a) wherein Y represents an integer between 11 and 15.

6. Use as claimed in claim 2 wherein a compound of formula (I) is (2S,3S,4R)-1-(α-D-galactopyranosyloxy)-2-hexacosanoylamino-3,4-octadecanediol.

7. Use of a cell mixture for the manufacture of a medicament for the treatment of disease selected from the group consisting of autoimmune disease, graft rejection, graft versus host disease, and allergic disease,
wherein said cell mixture comprises dendritic cells presenting at least antigen fragment of an antigen which is presented against T cell in T cell dependent immune response inducing said disease, and dendritic cells presenting at least alpha-glycosylceramide derivative.

8. Use as claimed in claim 7, wherein the cell mixture comprises dendritic cells presenting said antigen fragment and dendritic cells presenting alpha-glycosylceramide derivative.

9. Use as claimed in claim 7, wherein the cell mixture comprises dendritic cells presenting said antigen fragment and/or dendritic cells presenting alpha-glycosylceramide derivative, and dendritic cells presenting said antigen fragment and alpha-glycosylceramide derivative.

10. Use as claimed in claim 7, wherein the alpha-glycosylceramide derivative is a compound of formula (I), or a salt or a solvate thereof: 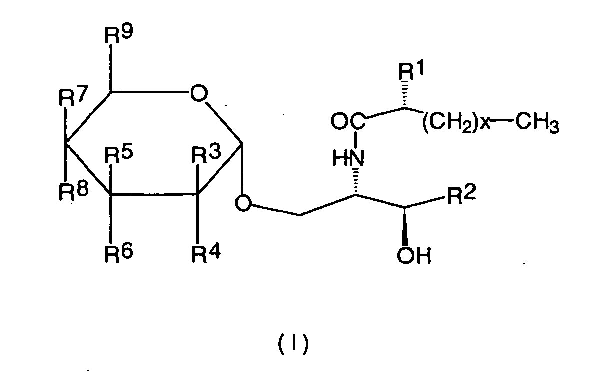 wherein R¹ to R⁹ and X are as defined in claim 2.

11. Use as claimed in claim 10 wherein R³ and R⁶ represent H, R⁴ represents OH or a substituent of any one of groups (A) to (D), R⁵ represents OH or a substituent of group (E) or (F), R⁷ and R⁸ each represent H or OH wherein both R⁷ and R⁸ do not represent the same substituent, and R⁹ represents CH₂OH, CH₃, H or a substituent of any one of groups (A') to (D').

12. Use as claimed in claim 10 wherein X represents an integer between 21 and 25 and R² represents substituent (b) wherein Y represents an integer between 11 and 15.

13. Use as claimed in claim 10 wherein X represents an integer between 9 and 13 and R² represents substituent (a) wherein Y represents an integer between 11 and 15.

14. Use as claimed in claim 10 wherein a compound of formula (I) is (2S,3S,4R)-1-(α-D-galactopyranosyloxy)-2-hexacosanoylamino-3,4-octadecanediol.

15. Use of a combination of dendritic cells for the manufacture of a medicament for the treatment of disease selected from the group consisting of autoimmune disease, graft rejection, graft versus host disease, and allergic disease,
wherein said combination consists of dendritic cells presenting at least antigen fragment of an antigen which is presented against T cell in T cell dependent immune response inducing said disease, and dendritic cells presenting at least alpha-glycosylceramide derivative.

16. Use as claimed in claim 15 wherein the combination consists of dendritic cells presenting said antigen fragment and dendritic cells presenting alpha-glycosylceramide derivative.

17. Use as claimed in claim 15 wherein the combination consists of dendritic cells presenting said antigen fragment and/or dendritic cells presenting alpha-glycosylceramide derivative, and dendritic cells presenting said antigen fragment and alpha-glycosylceramide derivative.

18. Use as claimed in claim 15 wherein the alpha-glycosylceramide derivative is a compound of formula (I), or a salt or a solvate thereof: 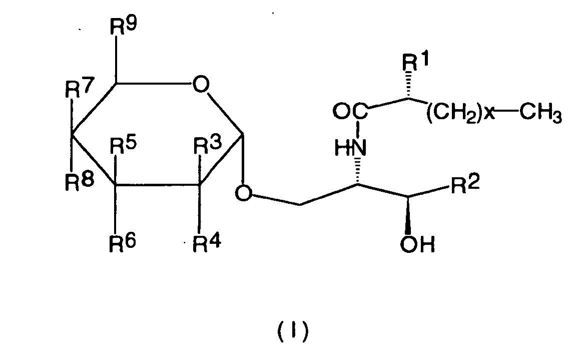 wherein R¹ to R⁹ and X are as defined in claim 2.

19. Use as claimed in claim 18 wherein R³ and R⁶ represent H, R⁴ represents OH or a substituent of any one of groups (A) to (D), R⁵ represents OH or a substituent of group (E) or (F), R⁷ and R⁸ each represent H or OH wherein both R⁷ and R⁸ do not represent the same substituent, and R⁹ represents CH₂OH, CH₃, H or a substituent of any one of groups (A') to (D').

20. Use as claimed in claim 18 wherein X represents an integer between 21 and 25 and R² represents substituent (b)
wherein Y represents an integer between 11 and 15.

21. Use as claimed in claim 18 wherein X represents an integer between 9 and 13 and R² represents substituent (a) wherein Y represents an integer between 11 and 15.

22. Use as claimed in claim 18 wherein a compound of formula (I) is (2S,3S,4R)-1-(α-D-galactopyranosyloxy)-2-hexacosanoylamino-3,4-octadecanediol.

## Patentansprüche

1. Verwendung einer dendritischen Zelle zur Herstellung eines Medikaments zur Behandlung einer Erkrankung, ausgewählt aus der Gruppe bestehend aus Autoimmunerkrankung, Transplantatabstoßung, Transplantat-versus-Wirt-Erkrankung und allergischer Erkrankung,
worin die dendritische Zelle ein Antigenfragment eines Antigens präsentiert, welches einer T-Zelle in einer T-Zell-abhängigen Immunantwort, die diese Erkrankung auslöst, präsentiert wird, und alpha-Glycosylceramidderivat.

2. Verwendung wie in Anspruch 1 beansprucht, worin das alpha-Glycosylceramidderivat eine Verbindung der Formel (I) ist: 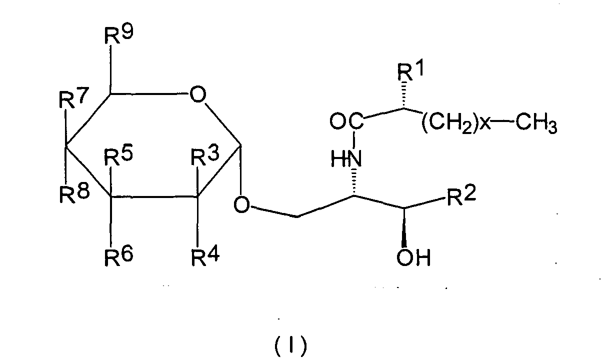 worin
R¹ H oder OH darstellt,
X eine ganze Zahl zwischen 7 und 27 darstellt,
R² einen Substituenten ausgewählt aus der Gruppe, die aus den folgenden (a) bis (e) besteht (worin Y eine ganze Zahl zwischen 5 und 17 ist), darstellt:
(a) -CH₂(CH₂)_{Y}CH₃
(b) -CH(OH)(CH₂)_{Y}CH₃
(c) -CH(OH)(CH₂)_{Y}CH(CH₃)₂
(d) -CH=CH(CH₂)_{Y}CH₃
(e) -CH(OH)(CH₂)_{Y}CH(CH₃)CH₂CH₃, und
R³ bis R⁹ Substituenten darstellen, die in den folgenden i) oder ii) definiert sind:
i) wenn R³, R⁶ und R⁸ H darstellen, stellt R⁴ H, OH, NH₂, NHCOCH₃ oder einen Substituenten dar, der aus der Gruppe ausgewählt ist, die aus den folgenden Gruppen (A) bis (D) besteht: 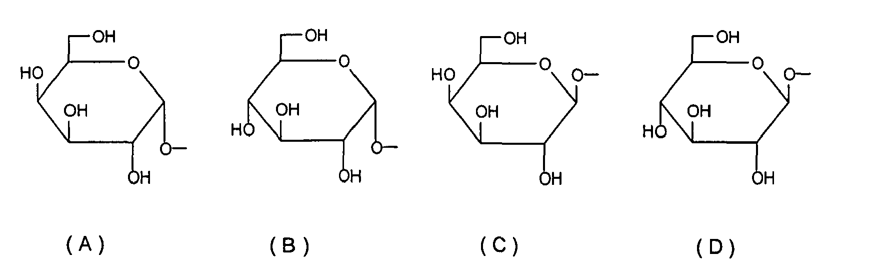 R⁵ OH oder einen Substituenten darstellt, der aus der Gruppe ausgewählt ist, die aus den folgenden Gruppen (E) und (F) besteht: 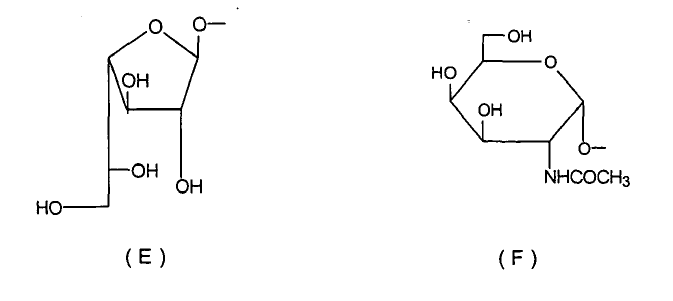 R⁷ OH oder einen Substituenten darstellt, der aus der Gruppe ausgewählt ist, die aus den folgenden Gruppen (A) bis (D) besteht: 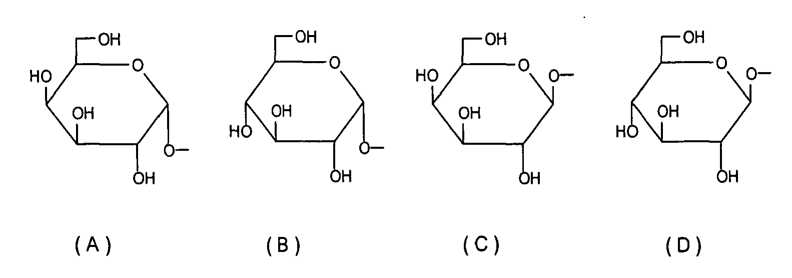 R⁹ H, CH₃, CH₂OH oder einen Substituenten darstellt, der aus der Gruppe ausgewählt ist, die aus den folgenden Gruppen (A') bis (D') besteht: 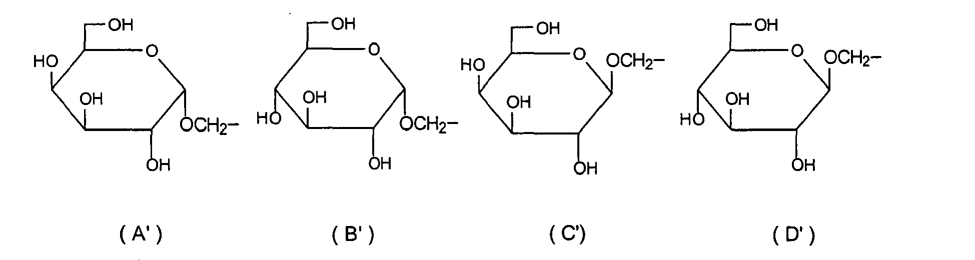
ii) wenn R³, R⁶ und R⁷ H darstellen, stellt R⁴ H, OH, NH₂, NHCOCH₃ oder einen Substituenten dar, der aus der Gruppe ausgewählt ist, die aus den folgenden Gruppen (A) bis (D) besteht: 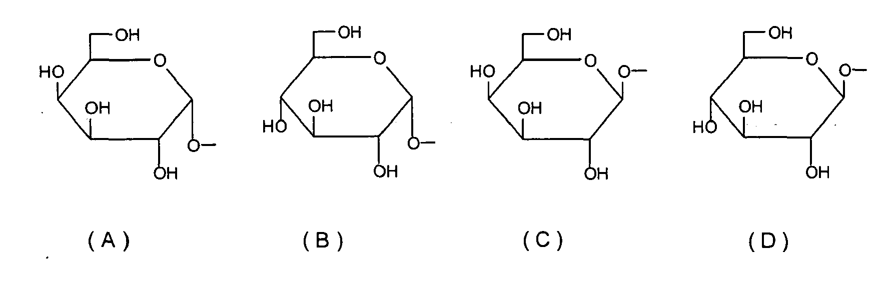 R⁵ OH oder einen Substituenten darstellt, der aus der Gruppe ausgewählt ist, die aus den folgenden Gruppen (E) und (F) besteht: 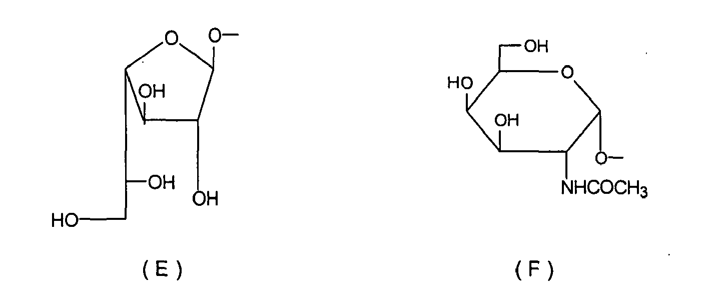 R⁸ OH oder einen Substituenten darstellt, der aus der Gruppe ausgewählt ist, die aus den folgenden Gruppen (A) bis (D) besteht: 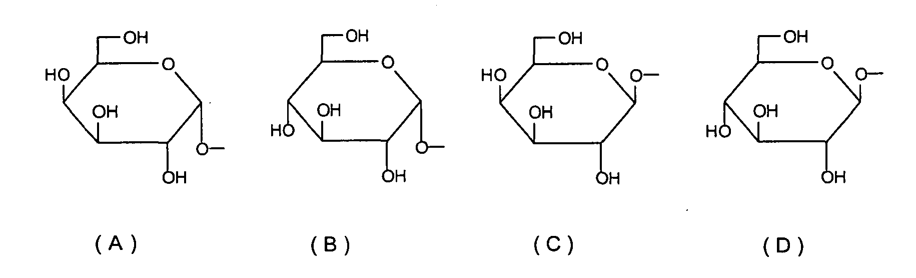 R⁹ H, CH₃, CH₂OH oder einen Substituenten darstellt, der aus der Gruppe ausgewählt ist, die aus den folgenden Gruppen (A') bis (D') besteht: 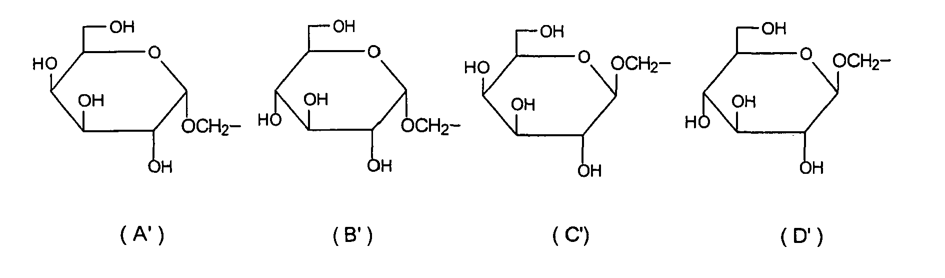
oder ein Salz oder ein Solvat davon.

3. Verwendung wie in Anspruch 2 beansprucht, worin R³ und R⁶ H darstellen, R⁴ OH oder einen Substituenten irgendeiner der Gruppen (A) bis (D) darstellt, R⁵ OH oder einen Substituenten der Gruppen (E) oder (F) darstellt, R⁷ und R⁸ jeweils H oder OH darstellen, wobei sowohl R⁷ als auch R⁸ nicht den gleichen Substituenten darstellen, und R⁹ CH₂OH, CH₃, H oder einen Substituenten irgendeiner der Gruppen (A') bis (D') darstellt.

4. Verwendung wie in Anspruch 2 beansprucht, worin X eine ganze Zahl zwischen 21 und 25 darstellt und R² den Substituenten (b) darstellt, worin Y eine ganze Zahl zwischen 11 und 15 darstellt.

5. Verwendung wie in Anspruch 2 beansprucht, worin X eine ganze Zahl zwischen 9 und 13 darstellt und R² den Substituenten (a) darstellt, worin Y eine ganze Zahl zwischen 11 und 15 darstellt.

6. Verwendung wie in Anspruch 2 beansprucht, wobei eine Verbindung der Formel (I) (2S,3S,4R)-1-(α-D-Galactopyranosyloxy)-2-hexacosanoylamino-3,4-octadecandiol ist.

7. Verwendung einer Zellmischung zur Herstellung eines Medikaments zur Behandlung einer Erkrankung, ausgewählt aus der Gruppe bestehend aus Autoimmunerkrankung, Transplantatabstoßung, Transplantat-versus-Wirt-Erkrankung und allergischer Erkrankung,
worin diese Zellmischung dendritische Zellen umfaßt, die mindestens ein Antigenfragment eines Antigens präsentieren, welches einer T-Zelle in einer T-Zell-abhängigen Immunantwort, die diese Erkrankung auslöst, präsentiert wird, und dendritischen Zellen, die mindestens alpha-Glycosylceramidderivat präsentieren.

8. Verwendung wie in Anspruch 7 beansprucht, wobei die Zellmischung dendritische Zellen umfaßt, die dieses Antigenfragment präsentieren, und dendritische Zellen, die alpha-Glycosylceramidderivat präsentieren.

9. Verwendung wie in Anspruch 7 beansprucht, wobei die Zellmischung dendritische Zellen, die dieses Antigenfragment präsentieren, und/oder dendritische Zellen, die alpha-Glycosylceramidderivat präsentieren, und dendritische Zellen, die das Antigenfragment und alpha-Glycosylceramidderivat präsentieren, umfaßt.

10. Verwendung wie in Anspruch 7 beansprucht, wobei das alpha-Glycosylceramidderivat eine Verbindung der Formel (I) oder ein Salz oder ein Solvat davon ist: 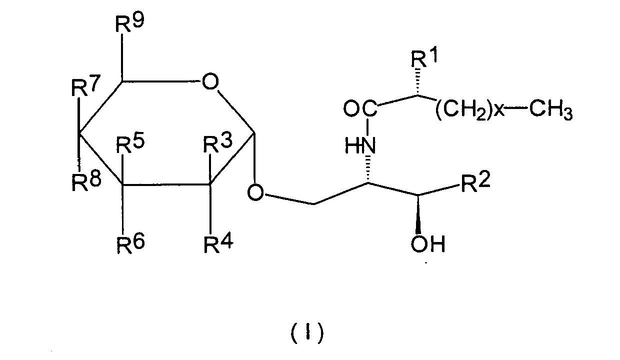 wobei R¹ bis R⁹ und X wie in Anspruch 2 definiert sind.

11. Verwendung wie in Anspruch 10 beansprucht, worin R³ und R⁶ H darstellen, R⁴ OH oder einen Substituenten irgendeiner der Gruppen (A) bis (D) darstellt, R⁵ OH oder einen Substituenten der Gruppen (E) oder (F) darstellt, R⁷ und R⁸ jeweils H oder OH darstellen, wobei sowohl R⁷ als auch R⁸ nicht den gleichen Substituenten darstellen, und R⁹ CH₂OH, CH₃, H oder einen Substituenten irgendeiner der Gruppen (A') bis (D') darstellt.

12. Verwendung wie in Anspruch 10 beansprucht, worin X eine ganze Zahl zwischen 21 und 25 darstellt und R² den Substituenten (b) darstellt, worin Y eine ganze Zahl zwischen 11 und 15 darstellt.

13. Verwendung wie in Anspruch 10 beansprucht, worin X eine ganze Zahl zwischen 9 und 13 darstellt und R² den Substituenten (a) darstellt, worin Y eine ganze Zahl zwischen 11 und 15 darstellt.

14. Verwendung wie in Anspruch 10 beansprucht, wobei eine Verbindung der Formel (I) (2S,3S,4R)-1-(α-D-Galactopyranosyloxy)-2-hexacosanoylamino-3,4-octadecandiol ist.

15. Verwendung einer Kombination aus dendritischen Zellen zur Herstellung eines Medikaments zur Behandlung von Erkrankungen, ausgewählt aus der Gruppe bestehend aus Autoimmunerkrankung, Transplantatabstoßung, Transplantat-versus-Wirt-Erkrankung und allergischer Erkrankung,
worin diese Kombination aus dendritischen Zellen besteht, die mindestens ein Antigenfragment eines Antigens präsentiert, welches einer T-Zelle in einer T-Zell-abhängigen Immunantwort, die diese Erkrankung auslöst, präsentiert wird, und dendritischen Zellen, die mindestens alpha-Glycosylceramidderivat präsentieren.

16. Verwendung wie in Anspruch 15 beansprucht, worin die Kombination aus dendritischen Zellen, die dieses Antigenfragment präsentieren, und dendritischen Zellen, die das alpha-Glycosylceramidderivat präsentieren, besteht.

17. Verwendung wie in Anspruch 15 beansprucht, worin die Kombination aus dendritischen Zellen, die dieses Antigenfragment präsentieren, und/oder dendritischen Zellen, die das alpha-Glycosylceramidderivat präsentieren, besteht, und die dendritischen Zellen dieses Antigenfragment und alpha-Glycosylceramidderivat präsentieren.

18. Verwendung wie in Anspruch 15 beansprucht, worin das alpha-Glycosylceramidderivat eine Verbindung der Formel (I) oder ein Salz oder Solvat davon ist: 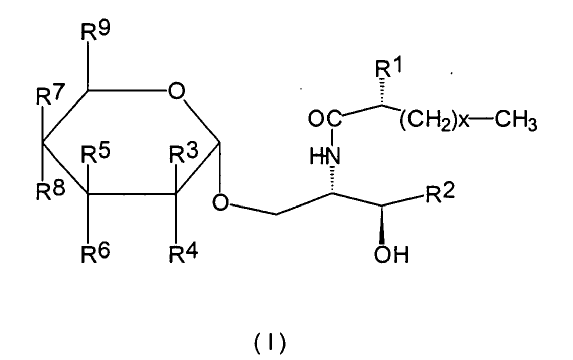 worin R¹ bis R⁹ und X wie in Anspruch 2 definiert sind.

19. Verwendung wie in Anspruch 18 beansprucht, worin R³ und R⁶ H darstellen, R⁴ OH oder einen Substituenten irgendeiner der Gruppen (A) bis (D) darstellt, R⁵ OH oder einen Substituenten der Gruppen (E) oder (F) darstellt, R⁷ und R⁸ jeweils H oder OH darstellen, wobei R⁷ und R⁸ nicht den gleichen Substituenten darstellen, und R⁹ CH₂OH, CH₃, H oder einen Substituenten irgendeiner der Gruppen (A') bis (D') darstellt.

20. Verwendung wie in Anspruch 18 beansprucht, worin X eine ganze Zahl zwischen 21 und 25 darstellt und R² den Substituenten (b) darstellt, worin Y eine ganze Zahl zwischen 11 und 15 darstellt.

21. Verwendung wie in Anspruch 18 beansprucht, worin X eine ganze Zahl zwischen 9 und 13 darstellt und R² den Substituenten (a) darstellt, worin Y eine ganze Zahl zwischen 11 und 15 darstellt.

22. Verwendung wie in Anspruch 18 beansprucht, worin eine Verbindung der Formel (I) (2S,3S,4R)-1-(α-D-Galactopyranosyloxy)-2-hexacosanoylamino-3,4-octadecandiol ist.

## Revendications

1. Utilisation d'une cellule dendritique pour la préparation d'un médicament destiné au traitement d'une maladie choisie dans le groupe constitué par une maladie autoimmune, un rejet de greffe, une maladie du greffon contre l'hôte, et une maladie allergique,
dans laquelle ladite cellule dendritique présente un fragment antigène d'un antigène qui est présenté contre les cellules T dans ladite maladie induisant une réponse immune dépendante des cellules T, et un dérivé alpha-glycosylcéramide.

2. Utilisation selon la revendication 1, dans laquelle le dérivé alpha-glycosylcéramide est un composé de formule (I): 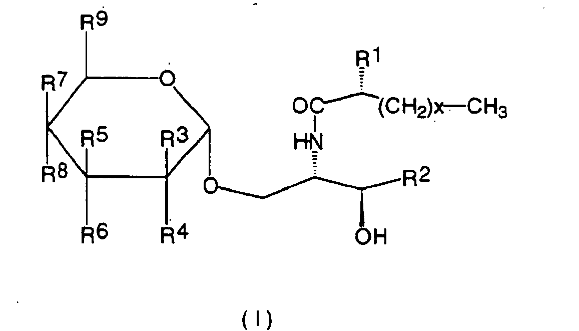 dans laquelle
R¹ représente H ou OH,
X représente un entier entre 7 et 27,
R² représente un substituant choisi dans le groupe constitué par les composés (a) à (e) suivants (où Y représente un entier entre 5 et 17) :
(a) -CH₂(CH₂)_{Y}CH₃
(b) -CH(OH)(CH₂)_{Y}CH₃
(c) -CH(OH)(CH₂)_{Y}CH(CH₃)₂
(d) -CH=CH(CH₂)_{Y}CH₃
(e) -CH(OH)(CH₂)_{Y}CH(CH₃)CH₂CH₃,
R³ à R⁹ représentent des substituants comme définis en i) ou ii) ci-dessous:
i) lorsque R³, R⁶ et R⁸ représentent H,
R⁴ représente H, OH, NH₂, NHCOCH₃, ou un substituant choisi dans le groupe comprenant les groupes (A) à (D) suivants: 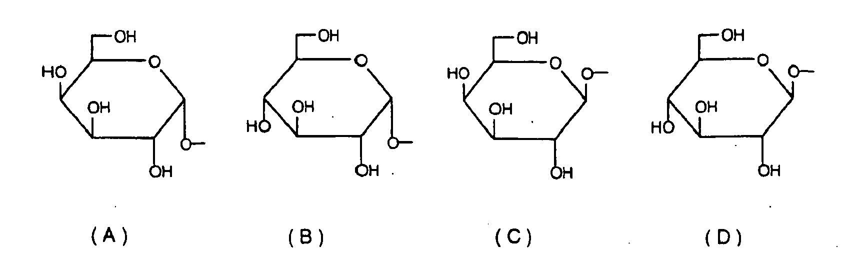
R₅ représente OH ou un substituant choisi dans le groupe comprenant les groupes (E) et (F) suivants : 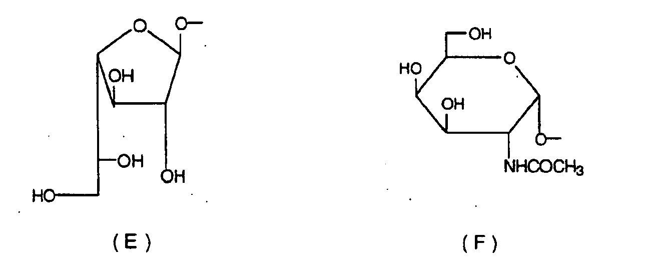
R⁷ représente OH ou un substituant choisi dans le groupe comprenant les groupes (A) à (D) suivants : 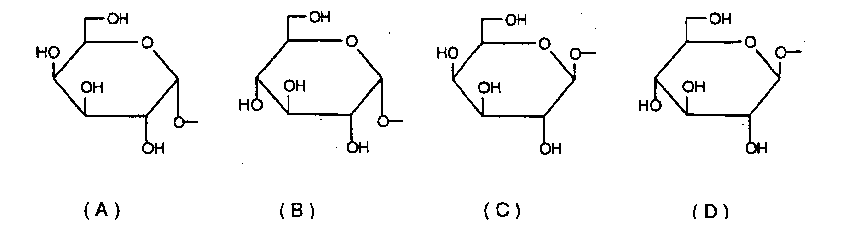
R⁹ représente H, CH₃, CH₂OH ou un substituant choisi dans le groupe comprenant les groupes (A') à (D') suivants : 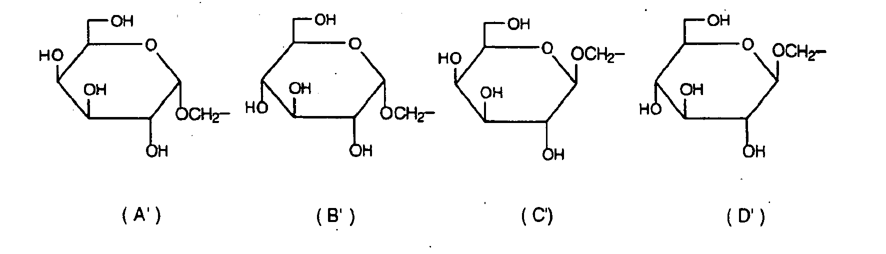
ii) lorsque R³, R⁶ et R⁷ représentent H,
R⁴ représente H, OH, NH₂, NHCOCH₃, ou un substituant choisi dans le groupe comprenant les groupes (A) à (D) suivants : 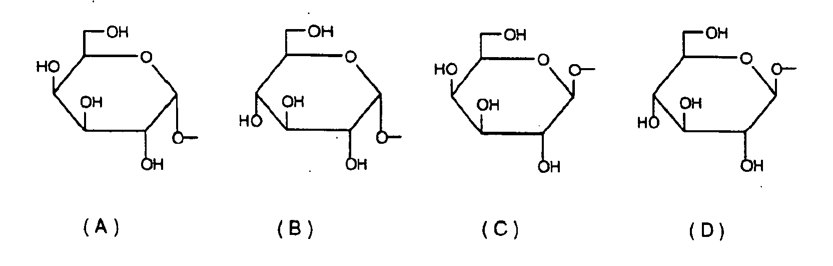
R₅ représente OH ou un substituant choisi dans le groupe comprenant les groupes (E) et (F) suivants : 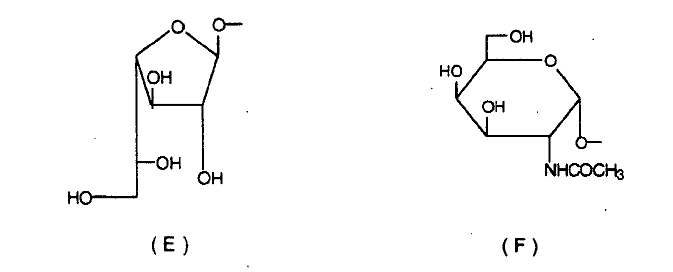
R⁸ représente OH ou un substituant choisi dans le groupe comprenant les groupes (A) à (D) suivants : 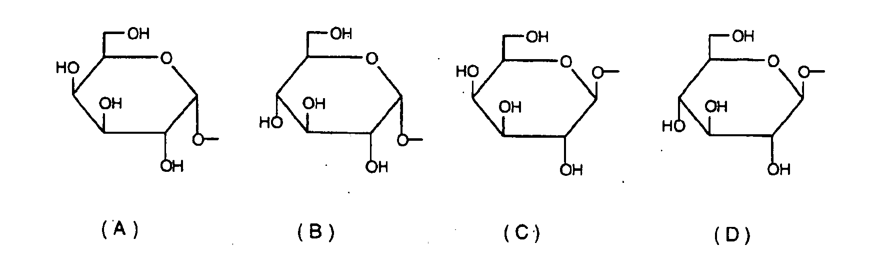
R⁹ représente H, CH₃, CH₂OH ou un substituant choisi dans le groupe comprenant les groupes (A') à (D') suivants : 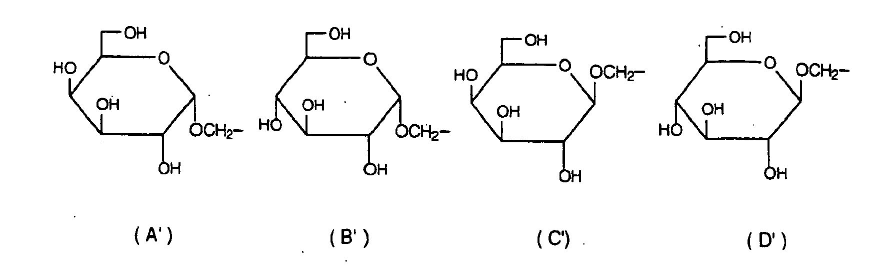
où un sel ou un solvate de celui-ci.

3. Utilisation selon la revendication 2, dans laquelle R³ et R⁶ représentent H, R⁴ représente OH ou un substituant choisi dans le groupe comprenant les groupes (A) à (D), R⁵ représente OH ou un substituant choisi dans le groupe (E) ou (F), R⁷ et R⁸ représentent chacun H ou OH, où R⁷ et R⁸ ne représentent pas le même substituant, et R⁹ représente H, CH₃, CH₂OH ou un substituant choisi dans le groupe comprenant les groupes (A') à (D').

4. Utilisation selon la revendication 2, dans laquelle X représente un entier entre 21 et 25 et R² représente le substituant (b) dans lequel Y représente un entier entre 11 et 15.

5. Utilisation selon la revendication 2, dans laquelle X représente un entier entre 9 et 13 et R² représente le substituant (a) dans lequel Y représente un entier entre 11 et 15.

6. Utilisation selon la revendication 2, dans laquelle un composé de formule (I) est le (2S,3S,4R)-1-(α-D-galactopyranosyloxy)-2-hexacosanoylamino-3,4-octadécanediol.

7. Utilisation d'un mélange de cellules pour la préparation d'un médicament destiné au traitement d'une maladie choisie dans le groupe constitué par une maladie autoimmune, un rejet de greffe, une maladie du greffon contre l'hôte, et une maladie allergique,
dans laquelle ledit mélange de cellules comprend des cellules dendritiques présentant au moins un fragment antigène d'un antigène qui est présenté contre les cellules T dans ladite maladie induisant une réponse immune dépendante des cellules T, et des cellules dendritiques présentant au moins un dérivé alpha-glycosylcéramide.

8. Utilisation selon la revendication 7, dans laquelle le mélange de cellules comprend des cellules dendritiques présentant ledit fragment antigène et des cellules dendritiques présentant un dérivé alpha-glycosylcéramide.

9. Utilisation selon la revendication 7, dans laquelle le mélange de cellules comprend des cellules dendritiques présentant ledit fragment antigène et/ou des cellules dendritiques présentant un dérivé alpha-glycosylcéramide, et des cellules dendritiques présentant ledit fragment antigène et un dérivé alpha-glycosylcéramide.

10. Utilisation selon la revendication 7, dans laquelle le dérivé alpha-glycosylcéramide est un composé de formule (I), ou un sel ou un solvate de celui-ci : 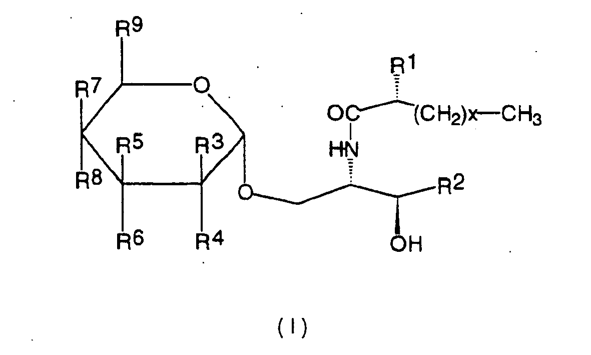 dans laquelle R¹ à R⁹ et X sont tels que définis dans la revendication 2.

11. Utilisation selon la revendication 10, dans laquelle R³ et R⁶ représentent H, R⁴ représente OH ou un substituant choisi dans le groupe comprenant les groupes (A) à (D), R⁵ représente OH ou un substituant choisi dans le groupe (E) ou (F), R⁷ et R⁸ représentent chacun H ou OH, où R⁷ et R⁸ ne représentent pas le même substituant, et R⁹ représente H, CH₃, CH₂OH ou un substituant choisi dans le groupe comprenant les groupes (A') à (D').

12. Utilisation selon la revendication 10, dans laquelle X représente un entier entre 21 et 25 et R² représente le substituant (b) dans lequel Y représente un entier entre 11 et 15.

13. Utilisation selon la revendication 10, dans laquelle X représente un entier entre 9 et 13 et R² représente le substituant (a) dans lequel Y représente un entier entre 11 et 15.

14. Utilisation selon la revendication 10, dans laquelle un composé de formule (I) est le (2S,3S,4R)-1-(α-D-galactopyranosyloxy)-2-hexacosanoylamino-3,4-octadécanediol.

15. Utilisation d'une combinaison de cellules dendritiques pour la préparation d'un médicament destiné au traitement d'une maladie choisie dans le groupe constitué par une maladie autoimmune, un rejet de greffe, une maladie du greffon contre l'hôte, et une maladie allergique,
dans laquelle ladite combinaison comprend des cellules dendritiques présentant au moins un fragment antigène d'un antigène qui est présenté contre les cellules T dans ladite maladie induisant une réponse immune dépendante des cellules T, et des cellules dendritiques présentant au moins un dérivé alpha-glycosylcéramide.

16. Utilisation selon la revendication 15, dans laquelle la combinaison comprend des cellules dendritiques présentant ledit fragment antigène et des cellules dendritiques présentant un dérivé alphaglycosylcéramide.

17. Utilisation selon la revendication 15, dans laquelle la combinaison comprend des cellules dendritiques présentant ledit fragment antigène et/ou des cellules dendritiques présentant un dérivé alpha-glycosylcéramide, et des cellules dendritiques présentant ledit fragment antigène et un dérivé alphaglycosylcéramide.

18. Utilisation selon la revendication 15, dans laquelle le dérivé alpha-glycosylcéramide est un composé de formule (I), ou un sel ou un solvate de celui-ci : 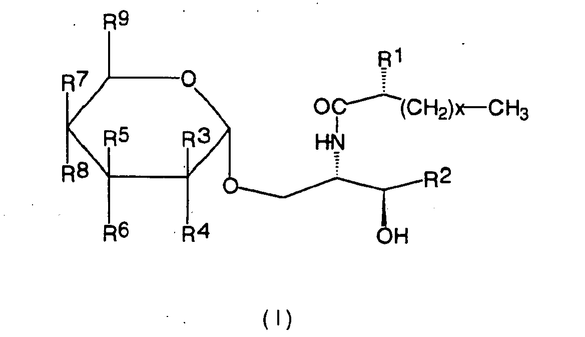 dans laquelle R¹ à R⁹ et X sont tels que définis dans la revendication 2.

19. Utilisation selon la revendication 18, dans laquelle R³ et R⁶ représentent H, R⁴ représente OH ou un substituant choisi dans le groupe comprenant les groupes (A) à (D), R⁵ représente OH ou un substituant choisi dans le groupe (E) ou (F), R⁷ et R⁸ représentent chacun H ou OH, où R⁷ et R⁸ ne représentent pas le même substituant, et R⁹ représente H, CH₃, CH₂OH ou un substituant choisi dans le groupe comprenant les groupes (A') à (D').

20. Utilisation selon la revendication 18, dans laquelle X représente un entier entre 21 et 25 et R² représente le substituant (b) dans lequel Y représente un entier entre 11 et 15.

21. Utilisation selon la revendication 18, dans laquelle X représente un entier entre 9 et 13 et R² représente le substituant (a) dans lequel Y représente un entier entre 11 et 15.

22. Utilisation selon la revendication 18, dans laquelle un composé de formule (I) est le (2S,3S,4R)-1-(α-D-galactopyranosyloxy)-2-hexacosanoylamino-3,4-octadécanediol.
